# EUROPEAN PATENT APPLICATION

(11) **EP 3 943 946 A1**
(43) Date of publication of application: **26.01.2022**
(21) Application number: 20186620.9
(22) Date of filing: 20.07.2020
(51) Int. Cl.: G01N 33/68

(54) **GDF-15 FOR PREDICTING THE DISEASE SEVERITY OF A PATIENT WITH COVID-19**

(71) Applicant: F. Hoffmann-La Roche AG, 4070 Basel (CH); Roche Diagnostics GmbH, 68305 Mannheim (DE)
(72) Inventor: OMLAND, Torbjoern, 1478 Nordbyhagen (NO); WIENHUES-THELEN, Ursula-Henrike, 82377 Penzberg (DE); ZIEGLER, Andre, 6343 Rotkreuz (CH)
(74) Representative: Wolters, Brit Kristin

(57) **Abstract**

The present invention relates to a method for predicting the disease severity in a patient with COVID-19, said method comprising a) determining the level of GDF-15 in a sample from the patient with COVID-19, b) comparing the level determined in step a) to a reference, and predicting the disease severity in a patient with COVID-19. Also encompassed are methods for risk stratification, monitoring of the disease progression and the risk prediction for hospital admission in a patient with COVID-19. Furthermore, methods are provided for predicting the need for intensive care, the risk for thrombosis, pulmonary embolism, hypoxia and mortality in a patient with COVID-19.

## Description

The present invention relates to a method for predicting the disease severity in a patient with COVID-19, said method comprising a) determining the level of GDF-15 in a sample from the patient with COVID-19, b) comparing the level determined in step a) to a reference, and predicting the disease severity in a patient with COVID-19.

Also encompassed are methods for risk stratification, monitoring of the disease progression and the risk prediction for hospital admission in a patient with COVID-19. Furthermore, methods are provided for predicting the need for intensive care, the risk for thrombosis, pulmonary embolism, hypoxia and mortality in a patient with COVID-19.

### Background

The SARS Corona-2 virus was discovered by Chinese virologists in the end of 2019 and has, since then, spread relentlessly throughout the world. Formerly known as nCoV-19 (novel Corona virus 2019), SARS CoV-2, the etiological agent of the Coronavirus Disease 2019 (COVID-19) triggered a pandemic in early 2020 leading to substantial restrictions of public life and severe economic effects worldwide.

Coronavirus disease 2019 (COVID-19) is caused by severe acute respiratory syndrome coronavirus 2 (SARS-CoV-2). The morbidity and mortality associated with the disease is high, and individuals with underlying cardiovascular disease (CVD) are disproportionately affected. Several studies have also noted higher incidences of cardiac arrhythmias, acute coronary syndromes and heart failure-related events among patients hospitalized with COVID-19, and cardiac injury is reported to contribute to cause of death in 40% of non-survivors. Also among survivors, a substantial proportion (20-30%) of hospitalized patients show evidence of cardiac injury during the infection (Guo T, et al., JAMA Cardiol. 2020. Guan W-j, et al., New England Journal of Medicine. 2020; 382:1708-1720. Zhou F, et al., The Lancet. 2020; 395:1054-1062. Shi S, et al. JAMA Cardiol. 2020, doi:10.1001/jamacardio.2020.0950. Ruan Q, et al., Intensive Care Med. 2020; 46:846-848).

The mechanisms linking SARS-CoV-2 to cardiac disease are yet unknown, though proposed hypotheses include cytokine storm, arterial hypoxia, hypoperfusion, coagulopathy, adrenergic stimulation and ACE-2 mediated injury (Varga Z, et al., Lancet (London, England). 2020;395:1417-1418). Vascular angiogenesis distinguished the pulmonary pathobiology of COVID-19 from that of equally severe influenza virus infection (Ackermann M et al., N Engl J Med. 2020 May 21. doi: 10.1056/NEJMoa2015432): Clinically, many patients had elevated d-dimer levels, as well as cutaneous changes in their extremities suggesting thrombotic microangiopathy. Diffuse intravascular coagulation and large-vessel thrombosis have been linked to multisystem organ failure. (Ackermann M et al., N Engl J Med. 2020 May 21. doi: 10.1056/NEJMoa2015432) It has been estimated that 10-20% of patients with COVID-19 have severe symptoms, so that knowledge of risk factors related to the aggravation of COVID-19 symptoms from asymptomatic or mild to life-threatening disease states helps in the patient management (Chang MC et al., BMC Infect Dis. 2020; 20: 445.)

Therefore, there is high need for biomarkers that provide prognostic information and predict the disease progression and severity for patients infected with SARS-CoV-2to assist in the efficient resource allocation in such pandemic disease, where symptoms can vary from mild to life-threatening. Peripheral oxygen saturation (SpO2) levels, concurrent comorbidities of COVID-19 patients, and additional risk factors have been suggested to determine the need for their admittance to ICUs. Biomarkers provide additional biological information on the affected tissues and mechanism of disease progression and thus serves as a basis for more targeted and intensified therapy stratification. (Velavan TP et al., Int J Infect Dis. 2020; 95: 304-307

To better differentiate the intensity and urgency of care needed in COVID-19, the severity of the disease was categorized by using the terms asymptomatic, mild, moderate, severe, and critical (Wang Y et al., J Med Virol 2020 Jun;92:568-576): Mild patients only present mild symptoms without radiographic features. Moderate patients present with fever, respiratory symptoms, and radiographic features. Severe patients meet one of three criteria: (a) dyspnea, RR greater than 30 times/min, (b) oxygen saturation less than 93% in ambient air, and (c) PaO2/FiO2 less than 300 mm Hg. Critical patients meet one of three criteria: (a) respiratory failure, (b) septic shock, and (c) multiple organ failure. (Wang Y et al., J Med Virol 2020 Jun;92:568-576). The largest epidemiology study done so far in China showed among 44,672 confirmed cases, that 80.9% were considered mild/common pneumonia, 13.8% were severe cases, 4.7% were critical cases, and an overall case-fatality rate of 2.3% (Epidemiology Working Group; Zhonghua Liu Xing Bing Xue Za Zhi 2020;41:145-151) suggesting that approximately 20% of the patients would need intensive care. The multiple organ failure in the category of critical COVID-19 patients suggest the need for markers of predicting early onset of multiple organ failure, where the biology of GDF-15 might fill that gap.

Growth differentiation factor 15 (GDF-15) is a member of the TGF beta family, it is synthesized as a 40 kD propeptide and undergoes cleavage of its N-terminal moiety to generate an active 30 kD disulfide-linked dimeric protein that is secreted. GDF-15 has been linked to heart failure and to cardiac reperfusion injury (Kempf T et al., 2006, Circulation Research, 98: 351-360). GDF-15 has been shown to be induced in many tissues in response to various stresses, such as ischemia, hypoxia, inflammation, tissue hypoperfusion, oxidative stress, mitochondrial dysfunctions, and neurohormonal activation. Release of GDF-15 can induce anti-inflammatory, anti-apoptotic and anti-proliferative effects, thereby exerting vasculoprotective mechanisms.(Altena R et al., PLoS One. 2015 Jan 15;10:e0115372) WO2008/015254 discloses a GDF-15 detection based method of identifying a patient with COVID-19 being eligible to a therapy of heart failure. Moreover, preferably a level of GDF- 15 (preferably 1200 pg/ml) larger than the reference is indicative for a patient with COVID-19 eligible to a therapy of heart failure.

It has been described that GDF-15 (also known as macrophage inhibitory cytokine-1) is a regulating factor in three pathophysiological conditions that are closely related: hypoxia, iron depletion, and inflammation. GDF-15 acts as a down-regulator of hepatic hepcidin which is the key regulator of iron handling (Finkenstedt A et al., Br J Haematol 2009;144:789-93). Inflammation has a potent effect on iron homeostasis, reducing intestinal iron absorption, and sequestering of iron in macrophages. Clinical studies found that levels of GDF15 were increased in patients with reduced tissue oxygenation which is believed to be due to the combination of ineffective erythropoiesis, anemia, hypercoagulability, and ischemia. (Lakal et al., BLOOD, 2009; 113, (7): 1555-1563) During profound hypoxia, tissue injury is caused by mitochondria degeneration. Anemic hypoxia induces general vasodilation, but also pulmonary vasoconstriction, with an increase of fibrin formation in lung microvasculature.(Cavezzi A et al., Clin Pract. 2020; doi: 10.4081/cp.2020.1271) GDF-15 is thus an early indicator of cellular oxygen deprivation of most organs released during mitochondrial dysfunction as sign of severe cellular injury.

Silent hypoxia in COVID-19 has been described as a sign of disease severity and a cause of poor outcomes (Kashani K et al., Mayo Clin Proc. 2020; 95(6):1094-1096). In these settings, silent hypoxia has been defined as the condition where the COVID-19 patient does not appear to be short of breath, or show an increase in respiratory rate, and not complaining of feeling air hunger, yet their blood oxygenation levels drop critically below 80%, which is an oxygen level considerably lower than normal. Arterial blood oxygen levels below 80 percent are sign of severe disease and may compromise function of most vital organs, such as brain and heart.

Physiological GDF-15 concentrations increase with age, while the expression in pathological states is highly regulated through several pathways including inflammation, oxidative stress and hypoxia. Elevated concentrations of circulating GDF-15 have been identified in multiple disease entities, i.e., CVD, sepsis, cancer and diabetes, and seem to be a robust predictor of disease progression. However, the role and prognostic importance of GDF-15 in COVID-19 is unknown. The half-life of GDF-15 under normal physiological conditions has been described as short as 3-hours (Xiong Y et al., Sci Transl Med 2017; 9:eaan8732). The incubation period for COVID-19 is reported with a median time of 4-5 days from exposure to symptoms onset, the time from onset of illness or symptoms to ICU admission approximately 10-12 days, and median length of hospitalization among survivors approximately 10 to 13 days. (Guan WJ et al., N Engl J Med 2020;382:1708-20). Based on the latest data available it has been proposed that patients who have had cardiac involvement initially should be seen every 1-3 months after the recovery of the acute phase of the disease. (Guzic TJ et al., Cardiovasc Res. 2020, doi: 10.1093/cvr/cvaa106)

Studies published from China early in the pandemic assessing inflammatory and cardiovascular biomarkers in patients with COVID-19 reported data from non-consecutive, retrospective series of hospitalized patients 2-5. These studies identified increasing interleukin-6 (IL-6), ferritin, cardiac troponin and D-dimer to be associated with worse outcome. Such study design introduces a substantial risk of selection bias, as the indication for measuring the biomarkers is determined by the treating physician.

The inventors aimed to investigate the associations between GDF-15 dynamics and cardiovascular and inflammatory biomarkers in a prospective study of patients infected with SARS-CoV-2.

Surprisingly, the inventors could show that GDF-15 is elevated in a high percentage of patients with COVID-19, and higher concentrations are associated with SARS-CoV-2 viremia and worse clinical outcome. GDF-15 provides prognostic information that is superior to known risk markers in COVID-19, including cTnT, NT-proBNP, CRP and D-dimer. Increased GDF-15 levels were inversely related to blood oxygenation levels. Greater increases in GDF-15 during hospitalization are also independently associated with worse outcomes.

### Summary of the Invention

In a first aspect, the present invention relates to a method for predicting the disease severity in a patient with COVID-19, said method comprising determining the level of GDF-15 in a sample from the patient with COVID-19,
   comparing the level determined in step a) to a reference, and predicting the disease severity in a patient with COVID-19.
In a second aspect, the present invention relates to a method for risk stratification of a patient with COVID-19, said method comprising
   a) determining the level of GDF-15 in a sample from the patient with COVID-19,
   b) comparing the level determined in step a) to a reference,
      stratifying the risk of a patient with COVID-19.
In a third aspect, the present invention relates to a method for monitoring disease progression in a patient with COVID-19, said method comprising
   a) determining the level of GDF-15 in a first sample from the patient with COVID-19,
   b) determining the level of GDF-15 in a second sample from the patient with COVID-19 which has been obtained after the first sample, and
   c) comparing the level of GDF-15 in the first sample to the level of GDF-15 in the second sample, and
   d) monitoring disease progression in a patient with COVID-19, based on the results of step c).
In a fourth aspect, the present invention relates to a method of predicting the risk in a patient with COVID-19 for hospital admission, said method comprising
   a) determining the level of GDF-15 in a sample from the patient with COVID-19,
   b) comparing the level determined in step a) to a reference,
   c) predicting the risk for hospital admission in a patient with COVID-19.
In a fifth aspect, the present invention relates to a method for prediction the need for intensive care of a patient with COVID-19, said method comprising
   a) determining the level of GDF-15 in a sample from the patient with COVID-19,
   b) comparing the level determined in step a) to a reference,
   c) predicting the need for intensive care of a patient with COVID-19.
In a sixth aspect, the present invention relates to a method for selecting a patient with COVID-19 for admission or treatment at Intensive Care Unit, said method comprising
   a) determining the level of GDF-15 in a sample from the patient with COVID-19,
   b) comparing the level determined in step a) to a reference,
   c) selecting a patient with COVID-19 for an Intensive Care Unit.
In a seventh aspect, the present invention relates to a method for predicting the risk for mortality in a patient with COVID-19, said method comprising
   a) determining the level of GDF-15 in a sample from the patient with COVID-19,
   b) comparing the level determined in step a) to a reference,
   c) predicting the risk for mortality in a patient with COVID-19.
In an eighth aspect, the present invention relates to an *in vitro* use of GDF-15 or of an agent which binds to GDF-15 for
   a) predicting the disease severity of a patient with COVID-19,
   b) risk stratification of a patient with COVID-19,
   c) monitoring the extent of the disease severity of a patient with COVID-19.
In a ninth aspect, the present invention relates to a method for predicting the risk for thrombosis in a patient with COVID-19, said method comprising
   a) determining the level of GDF-15 in a sample from the patient with COVID-19,
   b) comparing the level determined in step a) to a reference,
   c) predicting the risk for thrombosis of a patient with COVID-19.
In a tenth aspect, the present invention relates to a method for predicting the risk for pulmonary embolism of a patient with COVID-19, said method comprising
   a) determining the level of GDF-15 in a sample from the patient with COVID-19,
   b) comparing the level determined in step a) to a reference,
   c) predicting the risk for pulmonary embolism of a patient with COVID-19.
In an eleventh aspect, the present invention relates to a method for an *in vitro* use of GDF-15 or of an agent which binds to GDF-15 for
   a) predicting the disease severity of a patient with COVID-19,
   b) risk stratification of a patient with COVID-19,
   c) monitoring the extent of the disease severity of a patient with COVID-19.
In a twelfth aspect, the present invention relates to a method for an *in vitro* use of GDF-15 to diagnose COVID-19 patients with a
   a) decreased oxygen in blood (hypoxia),
   b) silent hypoxia, where the patient and physicians might not have been aware of the hypoxia and disease severity based on clinical symptoms,
   c) under perfusion (ischemia), insufficient oxygen supply and necrosis of tissue (generic hypoxia).

### List of Figures

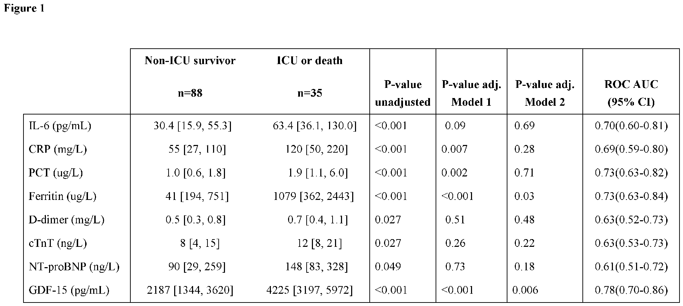

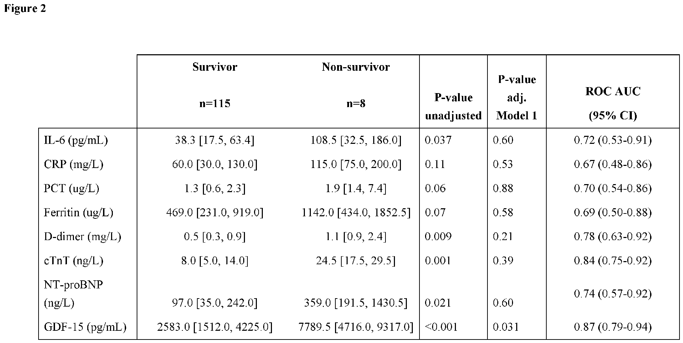

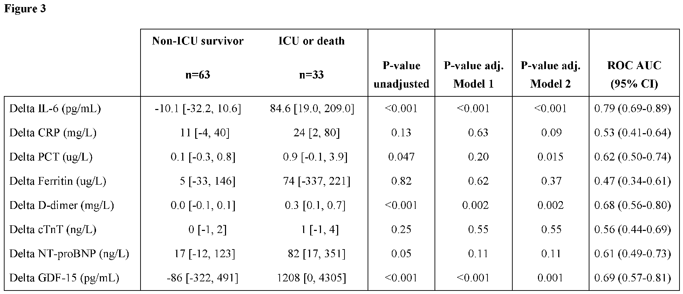

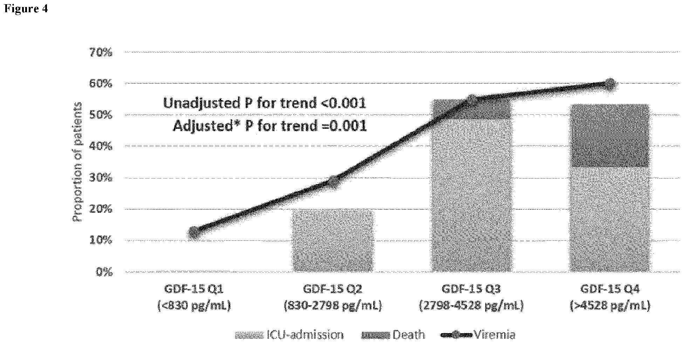

**Figure 1****:** Concentrations of admission cardiovascular and inflammatory biomarkers and the association with the primary endpoint among patients hospitalized with COVID-19 (n=123).
   Model 1: Adjusted for age, sex, race, body mass index, cardiovascular disease and creatinine
   Model 2: Adjusted for Model 1 + all the other biomarkers in the table Concentrations are reported as median (Q1, Q3). All biomarker values were log-transformed in the adjusted regression models.
   Abbreviations: IL-6= interleukin 6; CRP = C-reactive protein; PCT = procalcitonine; cTnT = cardiac troponin T; NT-proBNP = N-terminal pro-B-type natriuretic peptide; GDF-15= growth differentiation factor 15
**Figure 2****:** Concentrations of admission cardiovascular and inflammatory biomarkers and the association with all cause death among patients hospitalized with COVID-19 (n=123).
   Model 1: Adjusted for age, sex, race, body mass index, cardiovascular disease and creatinine
   Concentrations are reported as median (Q1, Q3). All biomarker values were log transformed in the adjusted regression model. Abbreviations: IL-6= interleukin 6; CRP = C-reactive protein; PCT = procalcitonine; cTnT = cardiac troponin T; NT-proBNP = N-terminal pro-B-type natriuretic peptide; GDF-15= growth differentiation factor 1
**Figure 3****:** Changes in concentrations from admission to day 3 in cardiovascular and inflammatory biomarkers stratified by the primary study endpoint among patients hospitalized with COVID-19 (n=96).
   Model 1: Adjusted for age, sex, race, body mass index, cardiovascular disease and creatinine
   Model 2: Adjusted for Model 1 + the baseline concentration of each biomarker Concentrations are reported as median (Q1, Q3). All biomarker delta values were log-transformed in the adjusted regression models.
   Abbreviations: IL-6= interleukin 6; CRP = C-reactive protein; PCT = procalcitonine; cTnT = cardiac troponin T; NT-proBNP = N-terminal pro-B-type natriuretic peptide; GDF-15= growth differentiation factor 15
**Figure 4****:** Proportion of patients with severe acute respiratory syndrome coronavirus 2 viremia at admission and proportion of patients reaching the primary endpoint by quartiles of growth differentiation factor 15 (GDF-15) admission concentrations. P-values are for trend in viremia across quartiles of GDF-15. * = Adjusted for age, sex, race, body mass index, cardiovascular disease and creatinine.

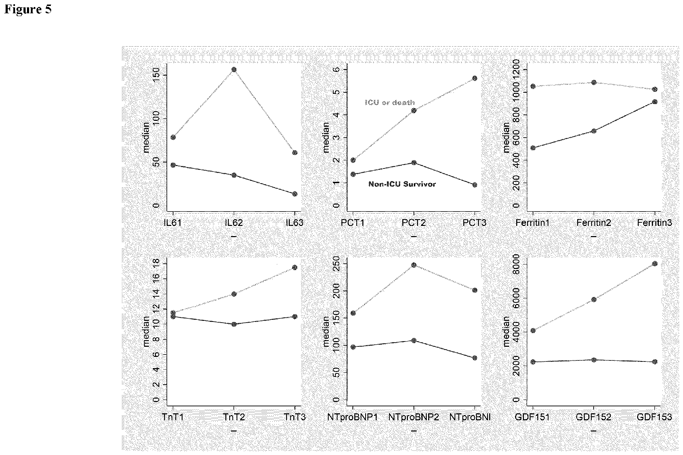

**Figure 5****:** Changes in interleukin-6, procalcitonin, ferritin, D-dimer, cardiac troponin T, N-terminal pro-B-type natriuretic peptide, and growth differentiation factor 15 (GDF-15) concentrations among SARS-CoV2 positive patients. Data from individuals with samples available from admission, on hospital day 3, and day 9, i.e. those hospitalized for ≥9 days (n=49). Values are median concentrations; groups are stratified by the composite primary endpoint, "ICU admission or death" versus "non-ICU-admitted survivors".

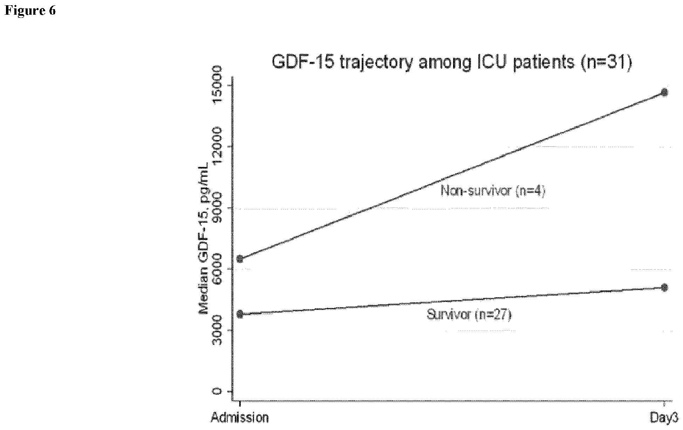

**Figure 6****:** Changes in GDF-15 concentrations among SARS-CoV-2 positive patients from admission to day 3 stratified by ICU survival. Values are median concentrations.

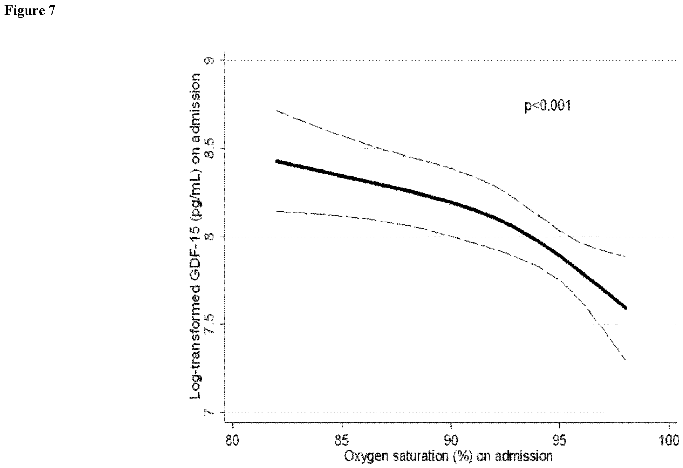

**Figure 7****:** Association between GDF-15 and oxygen saturation at baseline: There was a significant association between lower oxygen saturation and higher GDF-15: Beta coefficient -2.23 (95% CI -3.46 to -0.99) per log unit GDF-15, p=0.001. This association persisted after adjusting for age, sex, race, obesity (BMI ≥30 kg/m2), CVD (previous myocardial infarction, heart failure or atrial fibrillation) and creatinine.

**Table 1:** Baseline characteristics of the total COVID MECH study population (n=123) stratified by the primary study endpoint (admission to the intensive care unit or death).
**Table 2:** Baseline characteristics stratified by median growth differentiation factor 15 (GDF-15) concentration in the total population (2798 pg/ml).
**Table 3:** Multivariable regression of admission growth differentiation factor 15 concentrations.
**Table 4:** Spearman correlation between admission concentrations of biomarkers. All correlations were significant (p<0.05).
   Abbreviations: IL-6= interleukin 6; CRP = C-reactive protein; PCT = procalcitonine; cTnT = cardiac troponin T; NT-proBNP = N-terminal pro-B-type natriuretic peptide; GDF-15= growth differentiation factor 15.
**Table 5:** Multivariable logistic regression of severe acute respiratory syndrome coronavirus 2 viremia at admission, with all variables listed in the model.
**Table** 6: Changes in concentrations from admission to day 9 in GDF-15 stratified by ICU mortality among patients with COVID-19 (n=31) admitted to the ICU.

### Detailed Description of the Invention

Before the present invention is described in detail below, it is to be understood that this invention is not limited to the particular methodology, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art.

Several documents are cited throughout the text of this specification. Each of the documents cited herein (including all patents, patent applications, scientific publications, manufacturer's specifications, instructions etc.), whether supra or infra, is hereby incorporated by reference in its entirety. In the event of a conflict between the definitions or teachings of such incorporated references and definitions or teachings recited in the present specification, the text of the present specification takes precedence.

In the following, the elements of the present invention will be described. These elements are listed with specific embodiments, however, it should be understood that they may be combined in any manner and in any number to create additional embodiments. The various described examples and preferred embodiments should not be construed to limit the present invention to only the explicitly described embodiments. This description should be understood to support and encompass embodiments which combine the explicitly described embodiments with any number of the disclosed and/or preferred elements. Furthermore, any permutations and combinations of all described elements in this application should be considered disclosed by the description of the present application unless the context indicates otherwise.

The method of the present invention, preferably, is an ex vivo or in vitro method. Moreover, it may comprise steps in addition to those explicitly mentioned above. For example, further steps may relate to sample pre-treatments or evaluation of the results obtained by the method. The method may be carried out manually or assisted by automation. Preferably, step (a), (b) and/or (c) may in total or in part be assisted by automation, e.g., by a suitable robotic and sensory equipment for the determination in step (a), or a computer-implemented comparison and/or prediction based on said comparison in step (b).

### Definitions

The word "comprise", and variations such as "comprises" and "comprising", will be understood to imply the inclusion of a stated integer or step or group of integers or steps but not the exclusion of any other integer or step or group of integers or steps.

As used in this specification and the appended claims, the singular forms "a", "an", and "the" include plural referents, unless the content clearly dictates otherwise.

Levels, concentrations, amounts, and other numerical data may be expressed or presented herein in a "range" format. It is to be understood that such a range format is used merely for convenience and brevity and thus should be interpreted flexibly to include not only the numerical values explicitly recited as the limits of the range, but also to include all the individual numerical values or sub-ranges encompassed within that range as if each numerical value and sub-range is explicitly recited. As an illustration, a numerical range of "150 mg to 600 mg" should be interpreted to include not only the explicitly recited values of 150 mg to 600 mg, but to also include individual values and sub-ranges within the indicated range. Thus, included in this numerical range are individual values such as 150, 160, 170, 180, 190, ... 580, 590, 600 mg and sub-ranges such as from 150 to 200, 150 to 250, 250 to 300, 350 to 600, etc. This same principle applies to ranges reciting only one numerical value. Furthermore, such an interpretation should apply regardless of the breadth of the range or the characteristics being described.

The term "about" when used in connection with a numerical value is meant to encompass numerical values within a range having a lower limit that is 5% smaller than the indicated numerical value and having an upper limit that is 5% larger than the indicated numerical value.

The term "disease" and "disorder" are used interchangeably herein, referring to an abnormal condition, especially an abnormal medical condition such as an illness or injury, wherein a tissue, an organ or an individual is not able to efficiently fulfil its function anymore. Typically, but not necessarily, a disease is associated with specific symptoms or signs indicating the presence of such disease. The presence of such symptoms or signs may thus, be indicative for a tissue, an organ or an individual suffering from a disease. An alteration of these symptoms or signs may be indicative for the progression of such a disease. A progression of a disease is typically characterised by an increase or decrease of such symptoms or signs which may indicate a "worsening" or "bettering" of the disease. The "worsening" of a disease is characterised by a decreasing ability of a tissue, organ or organism to fulfil its function efficiently, whereas the "bettering" of a disease is typically characterised by an increase in the ability of a tissue, an organ or an individual to fulfil its function efficiently. Examples of a disease include but are not limited to infectious diseases, inflammatory diseases, cutaneous conditions, endocrine diseases, intestinal diseases, neurological disorders, joint diseases, genetic disorders, autoimmune diseases, traumatic diseases, and various types of cancer.

The term "disease severity" as used herein characterizes the impact that a disease process has on the utilization of resources, comorbidities, and mortality. The disease severity reflects the degree of illness and risk of disease manifested by patients, based either on clinical data from the medical records or on hospital discharge/billing data.

The term "disease progression" as used herein refers to the course of a disease. The term reflects a disease or physical ailment whose course in most cases is the worsening, growth, or spread of the disease. This may happen until death, serious debility, or organ failure occurs. Some progressive diseases can be halted and reversed by treatment. Many can be slowed by medical therapy. Some cannot be altered by current treatments. Diseases can be rapidly progressive (typically days to weeks) or slowly progressive (months to years). Virtually all slowly progressive diseases are also chronic diseases in terms of time course; many of these are also referred to as degenerative diseases. Not all chronic diseases are progressive: a chronic, non-progressive disease may be referred to as a static condition.

"Symptoms" of a disease are implication of the disease noticeable by the tissue, organ or organism having such disease and include but are not limited to pain, weakness, tenderness, strain, stiffness, and spasm of the tissue, an organ or an individual. "Signs" or "signals" of a disease include but are not limited to the change or alteration such as the presence, absence, increase or elevation, decrease or decline, of specific indicators such as biomarkers or molecular markers, or the development, presence, or worsening of symptoms. Symptoms of pain include, but are not limited to an unpleasant sensation that may be felt as a persistent or varying burning, throbbing, itching or stinging ache.

The term "Coronaviruses" refers to a group of related viruses that cause diseases in mammals and birds. In humans, Coronaviruses cause respiratory tract infections that can range from mild to lethal. Mild illnesses include some cases of the common cold, while more lethal varieties can cause "SARS", "MERS", and "COVID-19". Coronaviruses contain a positive-sense, single-stranded RNA genome.

The viral envelope is formed by a lipid bilayer wherein the membrane (M), envelope (E) and spike (S) structural proteins are anchored. Inside the envelope multiple copies of the nucleocapsid (N) protein form the nucleocapsid, which is bound to the positive-sense single-stranded RNA genome in a continuous beads-on-a-string type conformation. Its genome comprises Orfs 1a and 1b encoding the replicase/transcriptase polyprotein, followed by sequences encoding the spike (S)-envelope protein, the envelope (E)- protein, the membrane (M)-protein and the nucleocapsid (N)- protein. Interspersed between these reading frames are the reading frames for the accessory proteins which differ between the different virus strains.

Several human Coronaviruses are known, four of which lead to rather mild symptoms in patients:
Human Coronavirus NL63 (HCoV-NL63), α-CoV
Human Coronavirus 229E (HCoV-229E), α-CoV
Human Coronavirus HKU1 (HCoV-HKU1), β-CoV
Human Coronavirus OC43 (HCoV-OC43), β-CoV

Three human Coronaviruses produce symptoms that are potentially severe:
Middle East respiratory syndrome-related Coronavirus (MERS-CoV), β-CoV
Severe acute respiratory syndrome Coronavirus (SARS-CoV), β-CoV
Severe acute respiratory syndrome Coronavirus 2 (SARS-CoV-2), β-CoV

SARS-Cov-2 causes Coronavirus disease 2019 (COVID-19). Because the strain was first discovered in Wuhan, China, it is sometimes referred to as the Wuhan virus. SARS-Cov-2 is highly contagious in humans, and the World Health Organization (WHO) has designated the still ongoing pandemic of COVID-19 a Public Health Emergency of International Concern. The earliest case of infection currently known is thought to have been found on 17 November 2019. The SARS-Cov-2 sequence was first published on January 10, 2020 (Wuhan-Hu-1, GenBank accession number MN908947). Subsequent to the first outbreak in Wuhan, the virus spread to all provinces of China and to more than 150 other countries in Asia, Europe, North America, South America, Africa, and Oceania. Symptoms include high-fever, sore throat, dry cough, and exhaustion. In severe cases, pneumonia may develop.

The term "natural Corona virus" refers to a corona virus as occurring in nature, *i.e.* to any coronavirus as disclosed above. It is understood that a natural Corona virus comprises all proteins and nucleic acid molecules present in a naturally occurring virus. In difference to a natural Corona virus, "viral fragments", "virus-like particles", or Corona specific antigens, only comprise some but not all proteins and nucleic acid molecules present in a naturally occurring virus. Accordingly, such "viral fragments", "virus-like particles", or Corona specific antigens are not infectious but are still able to inflict an immune response in a patient. Accordingly, vaccination with Corona specific viral fragments, Corona specific virus-like particles, or Corona specific antigens inflicts the productions of antibodies against those viral fragments, virus-like particles, or antigens, in the patient.

The term "COVID_19" as used herein refers is an infectious disease caused by severe acute respiratory syndrome coronavirus 2 (SARS-CoV-2), formerly known as nCoV-19 (novel Corona virus 2019).

Coronaviruses (CoV) are large, enveloped, positive-sense RNA viruses. Human coronaviruses are typically associated with respiratory tract disease.

Coronaviruses that can infect humans, include human coronavirus (HCoV)-229E, HCoV-NL63, HCoV-HKU1, HCoV-OC43, MERS-CoV, SARS-CoV-1, and SARS-CoV-2.

Coronaviruses are divided in 3 genera: Alphacoronavirus, betacoronavirus, and gammacoronavirus. Betacoronaviruses SARS-CoV-2, SARS-CoV-1, and MERS-CoV are associated with severe disease in humans (Zubair, JAMA Neurology published online May 29, 2020, DOI: 10.1001/jamaneurol.2020.2065: Neuropathogenesis and Neurologic Manifestations of the Coronaviruses in the Age of Coronavirus Disease 2019 A Review).

The term "thrombosis" as use herein refers to the formation or presence of a blood clot in a blood vessel. The vessel may be any vein or artery as, for example, in a deep vein thrombosis or a coronary (artery) thrombosis. The clot itself is termed a thrombus. If the clot breaks loose and travels through the bloodstream, it is a thromboembolism.

The term "pulmonary embolism"(PE) is known in the art. Preferably, the term refers to blockage in a lung artery. It usually happens when a blood clot breaks loose and travels through the bloodstream to the lungs. PE is a serious condition that can cause the closure of a pulmonary artery or one of its branches, caused by a blood-borne clot or foreign material that plugs the vessel.

The term "Silent hypoxia" as used herein, has been defined as the condition where the patient does not appear to be short of breath, or show an increase in respiratory rate, and not complaining of feeling air hunger, yet their blood oxygenation levels drops critically below 80%, and thus an oxygen level lower than normal and considered to be severe: Arterial blood oxygen levels below 80 percent may compromise function of most vital organs, such as brain and heart. The term "Silent hypoxia" in patients with COVID-19 has been described as a sign of severe disease and cause for poor outcomes (Mayo Clin Proc. n June 2020; 95(6):1094-1096).

The term "hospital admission" or "hospitalization" is well understood by the skilled person and refers to the process of admitting a patient to a hospital involving a stay at hospital for at least one night or more. Staying in the hospital is done because the individuals are too sick to stay at home, require nursing care, and/or is receiving medications and undergoing tests and/or surgery that can only be performed in the hospital setting. Typically, but not necessarily a patient is admitted to hospital due to the development, presence, or progression of a disease. A patient may be admitted to hospital in cases where a mild form of a disease becomes more severe and more intense monitoring and/or treatment of the patient is required.

The term "intensive care unit" is known in the art. Preferably, the term refers to a health care unit that treats patients with life-threatening conditions which require constant support from equipment and medication in order to maintain normal bodily functions.

The term "mortality" as used herein, refers to the state or condition of being mortal or being patient with COVID-19 to death. The mortality rate is the percentage of deaths associated with a disease or medical treatment.

Preferably, the term "therapy" as used in the context of the method for predicting the risk of a patient with COVID-19 of hospitalization due to heart failure encompasses the estimation of probability of heart failure deterioration and worsening of symptoms in order to either prevent the disease deterioration altogether or significantly decrease its impact upon the patient, such as by preventing mortality or limiting morbidity.

The term "monitoring" as used herein, preferably, relates to assessing the disease progression as referred to herein elsewhere. Furthermore, the efficacy of a therapy for a COVID-19 patient may be monitored.

As used herein, a "patient" means any mammal, fish, reptile or bird that may benefit from the diagnosis, prognosis or treatment described herein. In particular, a "patient" is selected from the group consisting of laboratory animals (e.g. mouse, rat, rabbit, or zebrafish), domestic animals (including e.g. guinea pig, rabbit, horse, donkey, cow, sheep, goat, pig, chicken, camel, cat, dog, turtle, tortoise, snake, lizard or goldfish), or primates including chimpanzees, bonobos, gorillas and human beings. It is particularly preferred that the "patient" is a human being.

The term "sample" or "sample of interest" are used interchangeably herein, referring to a part or piece of a tissue, organ or individual, typically being smaller than such tissue, organ or individual, intended to represent the whole of the tissue, organ or individual. Upon analysis a sample provides information about the tissue status or the health or diseased status of an organ or individual. Samples of body fluids can be obtained by well-known techniques. Examples of samples include but are not limited to fluid samples such as blood, serum, plasma, synovial fluid, urine, saliva, sputum, ascites, lymphatic fluid, or solid samples such as tissue extracts, cartilage, bone, synovium, and connective tissue, or any other bodily secretion or derivative thereof. Tissue or organ samples may be obtained from any tissue or organ by, e.g., biopsy. Separated cells may be obtained from the body fluids or the tissues or organs by separating techniques such as centrifugation or cell sorting. E.g., cell-, tissue- or organ samples may be obtained from those cells, tissues or organs which express or produce the biomarker. The sample may be frozen, fresh, fixed (e.g. formalin fixed), centrifuged, and/or embedded (e.g. paraffin embedded), etc. The cell sample can, of course, be patient with COVID-19ed to a variety of well-known post-collection preparative and storage techniques (e.g., nucleic acid and/or protein extraction, fixation, storage, freezing, ultrafiltration, concentration, evaporation, centrifugation, etc.) prior to assessing the level of the biomarker(s) in the sample. Analysis of a sample may be accomplished on a visual or chemical basis. Visual analysis includes but is not limited to microscopic imaging or radiographic scanning of a tissue, organ or individual allowing for morphological evaluation of a sample. Chemical analysis includes but is not limited to the detection of the presence or absence of specific indicators or alterations in their amount, concentration or level. The sample is an in vitro sample, it will be analyzed in vitro and not transferred back into the body.

In a preferred embodiment of the present invention, the sample is a blood (i.e. whole blood), serum or plasma sample. Serum is the liquid fraction of whole blood that is obtained after the blood is allowed to clot. For obtaining the serum, the clot is removed by centrifugation and the supernatant is collected. Plasma is the acellular fluid portion of blood. For obtaining a plasma sample, whole blood is collected in anticoagulant-treated tubes (e.g. citrate-treated or EDTA-treated tubes). Cells are removed from the sample by centrifugation and the supernatant (i.e. the plasma sample) is obtained.

The term "reference" or "reference sample" or "control sample" as used herein, refers to a sample which is analysed in a substantially identical manner as the sample of interest and whose information is compared to that of the sample of interest. A reference sample thereby provides a standard allowing for the evaluation of the information obtained from the sample of interest. A control sample may be derived from a healthy or normal tissue, organ or individual, thereby providing a standard of a healthy status of a tissue, organ or individual. Differences between the status of the normal reference sample and the status of the sample of interest may be indicative of the risk of disease development or the presence or further progression of such disease or disorder. A control sample may be derived from an abnormal or diseased tissue, organ or individual thereby providing a standard of a diseased status of a tissue, organ or individual. Differences between the status of the abnormal reference sample and the status of the sample of interest may be indicative of a lowered risk of disease development or the absence or bettering of such disease or disorder. A reference sample may also be derived from the same tissue, organ, or individual as the sample of interest but has been taken at an earlier time point. Differences between the status of the earlier taken reference sample and the status of the sample of interest may be indicative of the progression of the disease, i.e. a bettering or worsening of the disease over time.

The control sample may be an internal or an external control sample. An internal control sample is used, i.e. the marker level(s) is (are) assessed in the test sample as well as in one or more other sample(s) taken from the same patient with COVID-19 to determine if there are any changes in the level(s) of said marker(s). For an external control sample the presence or level of a marker in a sample derived from the individual is compared to its presence or level in an individual known to suffer from, or known to be at risk of, a given condition; or an individual known to be free of a given condition, i.e., "normal individual".

It will be appreciated by the skilled artisan that such external control sample may obtained from a single individual or may be obtained from a reference population that is age-matched and free of confounding diseases. Typically, samples from 100 well-characterized individuals from the appropriate reference population are used to establish a "reference value". However, reference population may also be chosen to consist of 20, 30, 50, 200, 500 or 1000 individuals. Healthy individuals represent a preferred reference population for establishing a control value.

For example, a marker concentration in a patient sample can be compared to a concentration known to be associated with a specific course of a certain disease. Usually the sample's marker concentration is directly or indirectly correlated with a diagnosis and the marker concentration is e.g. used to determine whether an individual is at risk for a certain disease. Alternatively, the sample's marker concentration can e.g. be compared to a marker concentration known to be associated with a response to therapy in a certain disease, the diagnosis of a certain disease, the assessment of the severity of a certain disease, the guidance for selecting an appropriate drug to a certain disease, in judging the risk of disease progression, or in the follow-up of patients. Depending on the intended diagnostic use an appropriate control sample is chosen and a control or reference value for the marker established therein. As also clear to the skilled artisan, the absolute marker values established in a control sample will be dependent on the assay used.

The term "indicator" as used herein, refers to a sign or signal for a condition or is used to monitor a condition. Such a "condition" refers to the biological status of a cell, tissue or organ or to the health and/or disease status of an individual. An indicator may be the presence or absence of a molecule, including but not limited to peptide, protein, and nucleic acid, or may be a change in the expression level or pattern of such molecule in a cell, or tissue, organ or individual. An indicator may be a sign for the onset, development or presence of a disease in an individual or for the further progression of such disease. An indicator may also be a sign for the risk of developing a disease in an individual.

### Determination of the level of a biomarker

The terms "determining", "measuring", "measurement", "detecting" or "detection" are used herein interchangeably. The term "determining" the level of a biomarker as referred to herein refers to the quantification of the biomarker, e.g. to measuring the level of the biomarker in the sample, employing appropriate methods of detection described elsewhere herein.

In an embodiment, the determination of the level of a biomarker comprises by contacting the sample with an agent that specifically binds to the biomarker (such as an antibody of the present invention), thereby forming a complex between the agent and said biomarker, detecting the level of complex formed, and thereby measuring the level of said biomarker. The determination may further comprise steps, such as contacting the sample with a second agent.

The term "detecting" preferably comprises a qualitative, a semi-quantitative or a quantitative measurement. The term "detecting the presence" refers to a qualitative measurement, indicating the presence of absence without any statement to the quantities (e.g. yes or no statement).

The term "level" as used herein encompasses the absolute amount of a biomarker as referred to herein (such as one or more GDF-15 peptides), the relative amount or concentration of the said biomarker as well as any value or parameter which correlates thereto or can be derived therefrom. Such values or parameters comprise intensity signal values from all specific physical or chemical properties obtained from the said peptides by direct measurements, e.g., intensity values in mass spectra or NMR spectra. Moreover, encompassed are all values or parameters which are obtained by indirect measurements specified elsewhere in this description, e.g., response levels determined from biological read out systems in response to the peptides or intensity signals obtained from specifically bound ligands. It is to be understood that values correlating to the aforementioned levels or parameters can also be obtained by all standard mathematical operations.

The terms "lowered" or "decreased" level of an indicator refer to the level of such indicator in the sample being reduced in comparison to the reference or reference sample.

The terms "elevated" or "increased" level of an indicator refer to the level of such indicator in the sample being higher in comparison to the reference or reference sample. E.g. a protein that is detectable in higher levels in a fluid sample of one individual suffering from a given disease than in the same fluid sample of individuals not suffering from said disease, has an elevated level.

In the context of present invention, the term "biomarker" refers to a substance within a biological system that is used as an indicator of a biological state of said system. In the art, the term "biomarker" sometimes also applied to means for the detection of said endogenous substances (e.g. antibodies, nucleic acid probes etc, imaging systems). In the context of present invention, the term "biomarker"shall be only applied for the substance, not for the detection means. Thus, biomarkers can be any kind of molecule present in a living organism, such as a nucleic acid (DNA, mRNA, miRNA, rRNA etc.), a protein (cell surface receptor, cytosolic protein etc.), a metabolite or hormone (blood sugar, insulin, oestrogen, etc.), a molecule characteristic of a certain modification of another molecule (e.g. sugar moieties or phosphoryl residues on proteins, methyl-residues on genomic DNA) or a substance that has been internalized by the organism or a metabolite of such a substance.

The term "Growth-Differentiation Factor-15" or "GDF-15" relates to a polypeptide being a member of the transforming growth factor (TGF)-β cytokine superfamily. The terms polypeptide, peptide and protein are used interchangeable throughout this specification. GDF-15 is also called, MIC1, MIC-1, NAG-1, PDF, PLAB, PTGFB, Growth/differentiation factor 15, Placental bone morphogenetic protein, Placental TGF-beta, Macrophage inhibitory cytokine 1, Prostate differentiation factor, NSAID-activated gene 1 protein, NSAID-regulated gene 1 protein, NRG-1. GDF-15 was originally cloned as macrophage-inhibitory cytokine-1 and later also identified as placental transforming growth factor-β, placental bone morphogenetic protein, non-steroidal anti-inflammatory drug-activated gene-1, and prostate-derived factor (Bootcov loc cit; Hromas, 1997 Biochim Biophys Acta 1354:40-44; Lawton 1997, Gene 203:17-26; Yokoyama-Kobayashi 1997, J Biochem (Tokyo), 122:622-626; Paralkar 1998, J Biol Chem 273:13760-13767). Similar to other TGF-β-related cytokines, GDF-15 is synthesized as an inactive precursor protein, which undergoes disulfide-linked homodimerization. Upon proteolytic cleavage of the N-terminal pro-peptide, GDF-15 is secreted as a ∼28 kDa dimeric protein (Bauskin 2000, Embo J 19:2212-2220). Amino acid sequences and biological activities for GDF-15 are disclosed in WO99/06445, WO00/70051, WO2005/113585, Bottner 1999, Gene 237: 105-111, Bootcov loc. cit, Tan loc. cit., Baek 2001, Mol Pharmacol 59: 901-908, Hromas loc cit, Paralkar loc cit, Morrish 1996, Placenta 17:431-441 or Yokoyama-Kobayashi loc cit.. GDF-15 as used herein encompasses also variants of the aforementioned specific GDF-15 polypeptides. Such variants have at least the same essential biological and immunological properties as the specific GDF-15 polypeptides. In particular, they share the same essential biological and immunological properties if they are detectable by the same specific assays referred to in this specification, e.g., by ELISA assays using polyclonal or monoclonal antibodies specifically recognizing the said GDF-15 polypeptides. A preferred assay is described in the accompanying Examples. Moreover, it is to be understood that a variant as referred to in accordance with the present invention shall have an amino acid sequence which differs due to at least one amino acid substitution, deletion and/or addition wherein the amino acid sequence of the variant is still, preferably, at least about 50%, at least about 60%, at least about 70%, at least about 80%, at least about 85%, at least about 90%, at least about 92%, at least about 95%, at least about 97%, at least about 98%, or at least about 99% identical with the amino sequence of the specific GDF-15 polypeptides, preferably with the amino acid sequence of human GDF-15, more preferably over the entire length of the specific GDF-15, e.g. of human GDF-15. The degree of identity between two amino acid sequences can be determined as described above. Variants referred to above may be allelic variants or any other species specific homologs, paralogs, or orthologs. Moreover, the variants referred to herein include fragments of the specific GDF-15 polypeptides or the aforementioned types of variants as long as these fragments have the essential immunological and biological properties as referred to above. Such fragments may be, e.g., degradation products of the GDF-15 polypeptides. Further included are variants which differ due to posttranslational modifications such as phosphorylation or myristylation.

The biomarkers as referred to herein (such as the one or more GDF-15 peptides) can be detected using methods generally known in the art. Methods of detection generally encompass methods to quantify the level of a biomarker in the sample (quantitative method). It is generally known to the skilled artisan which of the following methods are suitable for qualitative and/or for quantitative detection of a biomarker. Samples can be conveniently assayed for, e.g., proteins using Westerns and immunoassays, like ELISAs, RIAs, fluorescence- and luminescence-based immunoassays and proximity extension assays, which are commercially available. Further suitable methods to detect biomarkers include measuring a physical or chemical property specific for the peptide or polypeptide such as its precise molecular mass or NMR spectrum. Said methods comprise, e.g., biosensors, optical devices coupled to immunoassays, biochips, analytical devices such as mass-spectrometers, NMR- analyzers, or chromatography devices. Further, methods include microplate ELISA-based methods, fully-automated or robotic immunoassays (available for example on Elecsys^{™} analyzers), CBA (an enzymatic Cobalt Binding Assay, available for example on Roche-Hitachi^{™} analyzers), and latex agglutination assays (available for example on Roche-Hitachi^{™} analyzers).

For the detection of biomarker proteins as referred to herein a wide range of immunoassay techniques using such an assay format are available, see, e.g., U.S. Pat. Nos. 4,016,043, 4,424,279, and 4,018,653. These include both single-site and two-site or "sandwich" assays of the non-competitive types, as well as in the traditional competitive binding assays. These assays also include direct binding of a labeled antibody to a target biomarker.

Methods employing electrochemiluminescent labels are well-known. Such methods make use of the ability of special metal complexes to achieve, by means of oxidation, an excited state from which they decay to ground state, emitting electrochemiluminescence. For review see Richter, M.M., Chem. Rev. 2004; 104: 3003-3036.

In an embodiment, the antibody (or an antigen-binding fragment thereof) to be used for measuring the level of a biomarker is ruthenylated or iridinylated. Accordingly, the antibody (or an antigen-binding fragment thereof) shall comprise a ruthenium label. In an embodiment, said ruthenium label is a bipyridine-ruthenium (II) complex. Alternatively the antibody (or an antigen-binding fragment thereof) shall comprise an iridium label. In an embodiment, said iridium label is a complex as disclosed in WO 2012/107419.

In an embodiment of the sandwich assay for the determination of the one or more GDF-15 peptides, the assay comprises a biotinylated first monoclonal antibody (or fragment thereof) and a ruthenylated second monoclonal antibody (or fragment thereof) that specifically binds one or more GDF-15. The two antibodies form sandwich immunoassay complexes with the one or more GDF-15 peptides in the sample.

Measuring the level of a polypeptide (such as a GDF-15) may, preferably, comprise the steps of (a) contacting the polypeptide with an agent that specifically binds said polypeptide, (b) (optionally) removing non-bound agent, (c) measuring the level of bound binding agent, i.e. the complex of the agent formed in step (a). According to a preferred embodiment, said steps of contacting, removing and measuring may be performed by an analyzer unit. According to some embodiments, said steps may be performed by a single analyzer unit of said system or by more than one analyzer unit in operable communication with each other. For example, according to a specific embodiment, said system disclosed herein may include a first analyzer unit for performing said steps of contacting and removing and a second analyzer unit, operably connected to said first analyzer unit by a transport unit (for example, a robotic arm), which performs said step of measuring.

The agent which specifically binds the biomarker (herein also referred to as "binding agent") may be coupled covalently or non-covalently to a label allowing detection and measurement of the bound agent. Labeling may be done by direct or indirect methods. Direct labeling involves coupling of the label directly (covalently or non-covalently) to the binding agent. Indirect labeling involves binding (covalently or non-covalently) of a secondary binding agent to the first binding agent. The secondary binding agent should specifically bind to the first binding agent. Said secondary binding agent may be coupled with a suitable label and/or be the target (receptor) of a tertiary binding agent binding to the secondary binding agent. Suitable secondary and higher order binding agents may include antibodies, secondary antibodies, and the well-known streptavidin-biotin system (Vector Laboratories, Inc.). The binding agent or substrate may also be "tagged" with one or more tags as known in the art. Such tags may then be targets for higher order binding agents. Suitable tags include biotin, digoxygenin, His-Tag, Glutathion-S-Transferase, FLAG, GFP, myc-tag, influenza A virus haemagglutinin (HA), maltose binding protein, and the like. In the case of a peptide or polypeptide, the tag is preferably at the N-terminus and/or C-terminus. Suitable labels are any labels detectable by an appropriate detection method. Typical labels include gold particles, latex beads, acridan ester, luminol, ruthenium complexes, iridium complexes, enzymatically active labels, radioactive labels, magnetic labels ("e.g. magnetic beads", including paramagnetic and superparamagnetic labels), and fluorescent labels. Enzymatically active labels include e.g. horseradish peroxidase, alkaline phosphatase, beta-Galactosidase, Luciferase, and derivatives thereof. Suitable substrates for detection include di-amino-benzidine (DAB), 3,3'-5,5'-tetramethylbenzidine, NBT-BCIP (4-nitro blue tetrazolium chloride and 5-bromo-4-chloro-3-indolyl-phosphate, avail-able as ready-made stock solution from Roche Diagnostics), CDP-Star^{™} (Amersham Bio-sciences), ECF^{™} (Amersham Biosciences). A suitable enzyme-substrate combination may result in a colored reaction product, fluorescence or chemoluminescence, which can be determined according to methods known in the art (e.g. using a light-sensitive film or a suit-able camera system). As for measuring the enzymatic reaction, the criteria given above apply analogously. Typical fluorescent labels include fluorescent proteins (such as GFP and its derivatives), Cy3, Cy5, Texas Red, Fluorescein, and the Alexa dyes (e.g. Alexa 568). Further fluorescent labels are available e.g. from Molecular Probes (Oregon). Also the use of quantum dots as fluorescent labels is contemplated. A radioactive label can be detected by any method known and appropriate, e.g. a light-sensitive film or a phosphor imager.

The level of a polypeptide may be, also preferably, determined as follows: (a) contacting a solid support comprising a binding agent for the polypeptide as described elsewhere herein with a sample comprising the peptide or polypeptide and (b) measuring the level of peptide or poly-peptide which is bound to the support. Materials for manufacturing supports are well-known in the art and include, inter alia, commercially available column materials, polystyrene beads, latex beads, magnetic beads, colloid metal particles, glass and/or silicon chips and surfaces, nitrocellulose strips, membranes, sheets, duracytes, wells and walls of reaction trays, plastic tubes etc.

In yet an aspect the sample is removed from the complex formed between the binding agent and the at least one marker prior to the measurement of the level of formed complex. Accordingly, in an aspect, the binding agent may be immobilized on a solid support. In yet an aspect, the sample can be removed from the formed complex on the solid support by applying a washing solution.

"Sandwich assays" are among the most useful and commonly used assays encompassing a number of variations of the sandwich assay technique. Briefly, in a typical assay, an unlabeled (capture) binding agent is immobilized or can be immobilized on a solid substrate, and the sample to be tested is brought into contact with the capture binding agent. After a suitable period of incubation, for a period of time sufficient to allow formation of a binding agent-biomarker complex, a second (detection) binding agent labeled with a reporter molecule capable of producing a detectable signal is then added and incubated, allowing time sufficient for the formation of another complex of binding agent-biomarker-labeled binding agent. Any unreacted material may be washed away, and the presence of the biomarker is determined by observation of a signal produced by the reporter molecule bound to the detection binding agent. The results may either be qualitative, by simple observation of a visible signal, or may be quantitated by comparison with a control sample containing known levels of biomarker.

The incubation steps of a typical sandwich assays can be varied as required and appropriate. Such variations include for example simultaneous incubations, in which two or more of binding agent and biomarker are co-incubated. For example, both, the sample to be analyzed and a labeled binding agent are added simultaneously to an immobilized capture binding agent. It is also possible to first incubate the sample to be analyzed and a labeled binding agent and to thereafter add an antibody bound to a solid phase or capable of binding to a solid phase.

The formed complex between a specific binding agent and the biomarker shall be proportional to the level of the biomarker present in the sample. It will be understood that the specificity and/or sensitivity of the binding agent to be applied defines the degree of proportion of at least one marker comprised in the sample which is capable of being specifically bound. Further details on how the measurement can be carried out are also found elsewhere herein. The level of formed complex shall be transformed into a level of the biomarker reflecting the level indeed present in the sample.

The terms "binding agent", "specific binding agent", "analyte-specific binding agent", "detection agent" and "agent that specifically binds to a biomarker" are used interchangeably herein. Preferably it relates to an agent that comprises a binding moiety which specifically binds the corresponding biomarker. Examples of "binding agents", "detection agents", "and agents" are a nucleic acid probe, nucleic acid primer, DNA molecule, RNA molecule, aptamer, antibody, antibody fragment, peptide, peptide nucleic acid (PNA) or chemical compound. A preferred agent is an antibody which specifically binds to the biomarker to be determined. The term "antibody" herein is used in the broadest sense and encompasses various antibody structures, including but not limited to monoclonal antibodies, polyclonal antibodies, multispecific antibodies (e.g., bispecific antibodies), and antibody fragments so long as they exhibit the desired antigen-binding activity (i.e. antigen-binding fragments thereof). Preferably, the antibody is a polyclonal antibody (or an antigen-binding fragment therefrom). More preferably, the antibody is a monoclonal antibody (or an antigen binding fragment therefore Moreover, as described elsewhere herein, it is envisaged that two monoclonal antibodies are used that bind at different positions of the one or more GDF-15 peptides (in a sandwich immunoassay). Thus, at least one antibody is used for the determination of the level of the GDF-15 peptide.

In an embodiment, the at least one antibody is a mouse monoclonal antibody. In another embodiment, the at least one antibody is a rabbit monoclonal antibody. In a further embodiment, the antibody is goat polyclonal antibody. In an even further embodiment, the antibody is a sheep polyclonal antibody.

The term "immunoglobulin (Ig)" as used herein refers to immunity conferring glycoproteins of the immunoglobulin superfamily. "Surface immunoglobulins" are attached to the membrane of effector cells by their transmembrane region and encompass molecules such as but not limited to B-cell receptors, T -cell receptors, class I and II major histocompatibility complex (MHC) proteins, beta-2 microglobulin (∼2M), CD3, CD4 and CDS.

Typically, the term "antibody" as used herein refers to secreted immunoglobulins which lack the transmembrane region and can thus, be released into the bloodstream and body cavities. Human antibodies are grouped into different isotypes based on the heavy chain they possess. There are five types of human Ig heavy chains denoted by the Greek letters: α, γ, δ, ε, and µ. The type of heavy chain present defines the class of antibody, *i.e.* these chains are found in IgA, IgD, IgE, IgG, and IgM antibodies, respectively, each performing different roles, and directing the appropriate immune response against different types of antigens. Distinct heavy chains differ in size and composition; and may comprise approximately 450 amino acids (Janeway et al. (2001) Immunobiology, Garland Science). IgA is found in mucosal areas, such as the gut, respiratory tract and urogenital tract, as well as in saliva, tears, and breast milk and prevents colonization by pathogens (Underdown & Schiff (1986) Annu. Rev. Immunol. 4:389-417). IgD mainly functions as an antigen receptor on B cells that have not been exposed to antigens and is involved in activating basophils and mast cells to produce antimicrobial factors (Geisberger et al. (2006) Immunology 118:429-437; Chen et al. (2009) Nat. Immunol. 10:889-898). IgE is involved in allergic reactions via its binding to allergens triggering the release of histamine from mast cells and basophils. IgE is also involved in protecting against parasitic worms (Pier et al. (2004) Immunology, Infection, and Immunity, ASM Press). IgG provides the majority of antibody-based immunity against invading pathogens and is the only antibody isotype capable of crossing the placenta to give passive immunity to fetus (Pier et al. (2004) Immunology, Infection, and Immunity, ASM Press). In humans there are four different IgG subclasses (IgGl, 2, 3, and 4), named in order of their abundance in serum with IgGl being the most abundant (∼66%), followed by IgG2 (∼23%), IgG3 (∼7%) and IgG (∼4%). The biological profile of the different IgG classes is determined by the structure of the respective hinge region. IgM is expressed on the surface of B cells in a monomeric form and in a secreted pentameric form with very high avidity. IgM is involved in eliminating pathogens in the early stages of B cell mediated (humoral) immunity before sufficient IgG is produced (Geisberger et al. (2006) Immunology 118:429-437). Antibodies are not only found as monomers but are also known to form dimers of two Ig units (e.g. IgA), tetramers of four Ig units (e.g. IgM of teleost fish), or pentamers of five Ig units (e.g. mammalian IgM).

Antibodies are typically made of four polypeptide chains comprising two identical heavy chains and identical two light chains which are connected via disulfide bonds and resemble a "Y"-shaped macro-molecule. Each of the chains comprises a number of immunoglobulin domains out of which some are constant domains and others are variable domains.

Immunoglobulin domains consist of a 2-layer sandwich of between 7 and 9 antiparallel ∼-strands arranged in two ∼-sheets. Typically, the heavy chain of an antibody comprises four Ig domains with three of them being constant (CH domains: CHI. CH2. CH3) domains and one of the being a variable domain (V H). The light chain typically comprises one constant Ig domain (CL) and one variable Ig domain (V L). Exemplified, the human IgG heavy chain is composed of four Ig domains linked from N- to C-terminus in the order VwCH1-CH2-CH3 (also referred to as VwCyl-Cy2-Cy3), whereas the human IgG light chain is composed of two immunoglobulin domains linked from N- to C-terminus in the order VL-CL, being either of the kappa or lambda type (VK-CK or VA.-CA.). Exemplified, the constant chain of human IgG comprises 447 amino acids. Throughout the present specification and claims, the numbering of the amino acid positions in an immunoglobulin are that of the "EU index" as in Kabat, E. A., Wu, T.T., Perry, H. M., Gottesman, K. S., and Foeller, C., (1991) Sequences of proteins of immunological interest, 5thed. U.S. Department of Health and Human Service, National Institutes of Health, Bethesda, MD. The "EU index as in Kabat" refers to the residue numbering of the human IgG lEU antibody. Accordingly, CH domains in the context of IgG are as follows: "CHI" refers to amino acid positions 118-220 according to the EU index as in Kabat; "CH2" refers to amino acid positions 237-340 according to the EU index as in Kabat; and "CH3" refers to amino acid positions 341-44 7 according to the EU index as in Kabat.

The term "binding affinity" generally refers to the strength of the sum total of noncovalent interactions between a single binding site of a molecule (e.g., an antibody) and its binding partner (e.g., an antigen). Unless indicated otherwise, as used herein, "binding affinity" refers to intrinsic binding affinity which reflects a 1:1 interaction between members of a binding pair (e.g., antibody and antigen). The affinity of a molecule X for its partner Y can generally be represented by the dissociation constant (Kd). Affinity can be measured by common methods known in the art, including but not limited to surface plasmon resonance based assay (such as the BIAcore assay as described in PCT Application Publication No. WO2005/012359); enzyme-linked immunoabsorbent assay (ELISA); and competition assays (e.g. RIA's). Low-affinity antibodies generally bind antigen slowly and tend to dissociate readily, whereas high-affinity antibodies generally bind antigen faster and tend to remain bound longer. A variety of methods of measuring binding affinity are known in the art, any of which can be used for purposes of the present invention.

The term "tag" refers to those effector groups which provide the antigen with the ability to bind to or to be bound to other molecules. Examples of tags include but are not limited to e.g. His tags which are attached to the antigen sequence to allow for its purification. Tag may also include a partner of a bioaffine binding pair which allows the antigen to be bound by the second partner of the binding pair. The term "bioaffine binding pair" refers to two partner molecules (i.e. two partners in one pair) having a strong affinity to bind to each other. Examples of partners of bioaffine binding pairs are a) biotin or biotin analogs / avidin or streptavidin; b) Haptens / anti-hapten antibodies or antibody fragments (e.g. digoxin / anti-digoxin antibodies); c) Saccharides / lectins; d) complementary oligonucleotide sequences (e.g. complementary LNA sequences), and in general e) ligands / receptors.

The term "label" refers to those effector groups which allow for the detection of the antigen. Label include but are not limited to spectroscopic, photochemical, biochemical, immunochemical, or chemical, label. Exemplified, suitable labels include fluorescent dyes, luminescent or electrochemiluminescent complexes (e.g. ruthenium or iridium complexes), electron-dense reagents, and enzymatic label.

A "particle" as used herein means a small, localized object to which can be ascribed a physical property such as volume, mass or average size. Particles may accordingly be of a symmetrical, globular, essentially globular or spherical shape, or be of an irregular, asymmetric shape or form. The size of a particle may vary. The term "microparticle" refers to particles with a diameter in the nanometer and micrometer range.

Microparticles as defined herein above may comprise or consist of any suitable material known to the person skilled in the art, e.g. they may comprise or consist of or essentially consist of inorganic or organic material. Typically, they may comprise or consist of or essentially consist of metal or an alloy of metals, or an organic material, or comprise or consist of or essentially consist of carbohydrate elements. Examples of envisaged material for microparticles include agarose, polystyrene, latex, polyvinyl alcohol, silica and ferromagnetic metals, alloys or composition materials. In one embodiment the microparticles are magnetic or ferromagnetic metals, alloys or compositions. In further embodiments, the material may have specific properties and e.g. be hydrophobic, or hydrophilic. Such microparticles typically are dispersed in aqueous solutions and retain a small negative surface charge keeping the microparticles separated and avoiding nonspecific clustering.

In one embodiment of the present invention, the microparticles are paramagnetic microparticles and the separation of such particles in the measurement method according to the present disclosure is facilitated by magnetic forces. Magnetic forces are applied to pull the paramagnetic or magnetic particles out of the solution/suspension and to retain them as desired while liquid of the solution/suspension can be removed and the particles can e.g. be washed.

A "kit" is any manufacture (e.g. a package or container) comprising at least one reagent, e.g., a medicament for treatment of a disorder, or a probe for specifically detecting a biomarker gene or protein of the invention. The kit is preferably promoted, distributed, or sold as a unit for performing the methods of the present invention. Typically, a kit may further comprise carrier means being compartmentalized to receive in close confinement one or more container means such as vials, tubes, and the like In particular, each of the container means comprises one of the separate elements to be used in the method of the present invention. A label may be present on the container to indicate that the composition is used for a specific application, and may also indicate directions for either in vivo or in vitro use. Kits may further comprise one or more other containers comprising further materials including but not limited to buffers, diluents, filters, needles, syringes, and package inserts with instructions for use. These instructions may be in the form of a manual or may be provided by a computer program code which is capable of carrying out the calculations and comparisons referred to in the methods of the present invention and to establish the assessment or diagnosis accordingly when implemented on a computer or a data processing device. The computer program code may be provided on a data storage medium or device such as an optical storage medium (e.g., a Compact Disc) or directly on a computer or data processing device. Moreover, the kit may, preferably, comprise standard levels for a GDF-15-type peptide for calibration purposes.

In a preferred embodiment, the kit further comprises standard levels for the at least one further biomarker as referred to herein (such as TnT, NT-proBNP, CRP and D-dimer) for calibration purposes.

A "package insert" is used to refer to instructions customarily included in commercial packages of therapeutic products or medicaments, that contain information about the indications, usage, dosage, administration, contraindications, other therapeutic products to be combined with the packaged product, and/or warnings concerning the use of such therapeutic products or medicaments, etc.

The term "comparing" as used herein refers to comparing the level of the biomarker (such as the one or more GFD-15 -type peptides) in the sample from the patient with COVID-19 with the reference of the biomarker specified elsewhere in this description. It is to be understood that comparing as used herein usually refers to a comparison of corresponding parameters or values, e.g., an absolute level is compared to an absolute reference level while a concentration is compared to a reference concentration or an intensity signal obtained from the biomarker in a sample is compared to the same type of intensity signal obtained from a first sample. The comparison may be carried out manually or computer-assisted. Thus, the comparison may be carried out by a computing device. The value of the determined or detected level of the biomarker in the sample from the patient with COVID-19 and the reference level can be, e.g., compared to each other and the said comparison can be automatically carried out by a computer program executing an algorithm for the comparison. The computer program carrying out the said evaluation will provide the desired assessment in a suitable output format. For a computer-assisted comparison, the value of the determined level may be compared to values corresponding to suitable references which are stored in a database by a computer program. The computer program may further evaluate the result of the comparison, i.e. automatically provide the desired assessment in a suitable output format. For a computer-assisted comparison, the value of the determined level may be compared to values corresponding to suitable references which are stored in a database by a computer program. The computer program may further evaluate the result of the comparison, i.e. automatically provides the desired assessment in a suitable output format.

In accordance with the present invention, the level of one or more GDF-15 peptides shall be compared to a reference. The reference is preferably a reference.

The term "reference" is well understood by the skilled person. It is to be understood that the reference shall allow for the herein described prediction of the disease severity and disease progression in a patient with COVID-19.

The term "disease progression" as used herein refers to the course of a disease. The term reflects a disease or physical ailment whose course in most cases is the worsening, growth, or spread of the disease. This may happen until death, serious debility, or organ failure occurs. Some progressive diseases can be halted and reversed by treatment. Many can be slowed by medical therapy. Some cannot be altered by current treatments. Diseases can be rapidly progressive (typically days to weeks) or slowly progressive (months to years). Virtually all slowly progressive diseases are also chronic diseases in terms of time course; many of these are also referred to as degenerative diseases. Not all chronic diseases are progressive: a chronic, non-progressive disease may be referred to as a static condition.

E.g., in connection with the method for predicting the disease severity, the reference preferably refers to a level which allows for allocation of a patient with COVID-19 into either (i) the group of patients with COVID-19 for which a high disease severity is predicted or (ii) the group of patients with COVID-19 for which a low disease severity is predicted. A suitable reference may be determined from a first sample to be analyzed together, i.e. simultaneously or subsequently, with the test sample.

References can, in principle, be calculated for a cohort of patient with COVID-19s as specified above based on the average or mean values for a given biomarker by applying standard methods of statistics. In particular, accuracy of a test such as a method aiming to diagnose an event, or not, is best described by its receiver-operating characteristics (ROC) (see especially Zweig MH. et al., Clin. Chem. 1993; 39:561-577). The ROC graph is a plot of all the sensitivity versus specificity pairs resulting from continuously varying the decision threshold over the entire range of data observed. The clinical performance of a diagnostic method depends on its accuracy, i.e. its ability to correctly allocate patient with COVID-19s to a certain prognosis or diagnosis. The ROC plot indicates the overlap between the two distributions by plotting the sensitivity versus 1 - specificity for the complete range of thresholds suitable for making a distinction. On the y-axis is sensitivity, or the true-positive fraction, which is defined as the ratio of number of true-positive test results to the product of number of true-positive and number of false-negative test results. It is calculated solely from the affected subgroup. On the x-axis is the false-positive fraction, or 1 - specificity, which is defined as the ratio of number of false-positive results to the product of number of true-negative and number of false-positive results. It is an index of specificity and is calculated entirely from the unaffected subgroup. Because the true- and false-positive fractions are calculated entirely separately, by using the test results from two different subgroups, the ROC plot is independent of the prevalence of the event in the cohort. Each point on the ROC plot represents a sensitivity/1 - specificity pair corresponding to a particular decision threshold. A test with perfect discrimination (no overlap in the two distributions of results) has an ROC plot that passes through the upper left corner, where the true-positive fraction is 1.0, or 100% (perfect sensitivity), and the false-positive fraction is 0 (perfect specificity). The theoretical plot for a test with no discrimination (identical distributions of results for the two groups) is a 45° diagonal line from the lower left corner to the upper right corner. Most plots fall in between these two extremes. If the ROC plot falls completely below the 45° diagonal, this is easily remedied by reversing the criterion for "positivity" from "greater than" to "less than" or vice versa. Qualitatively, the closer the plot is to the upper left corner, the higher the overall accuracy of the test. Dependent on a desired confidence interval, a threshold can be derived from the ROC curve allowing for the diagnosis for a given event with a proper balance of sensitivity and specificity, respectively. Accordingly, the reference to be used for the method of the present invention, i.e. a threshold which allows to assess atrial fibrillation can be generated, preferably, by establishing a ROC for said cohort as described above and deriving a threshold level therefrom. Dependent on a desired sensitivity and specificity for a diagnostic method, the ROC plot allows deriving a suitable threshold.

Especially for patients presenting with only mild symptoms to the hospitals, it will be understood that an optimal specificity is envisaged for a patient with COVID-19 to be selecting for admission or treatment at Intensive Care Unit (i.e. a rule in), whereas an optimal sensitivity is desired for e.g. excluding a patient with COVID-19 for admission or treatment at the Intensive Care Unit (i.e. a rule out) and discharge from the hospital after care.

The term "prognosis" as use herein refers to predicting the likely or expected development of a disease, including whether the signs and symptoms will improve or worsen and how quickly or remain stable over time. The term further encompasses the expectations of quality of life, such as the ability to carry out daily activities; the potential for complications and associated Definition health issues; and the likelihood of survival including life expectancy. A prognosis is made on the basis of the normal course of the diagnosed disease, the individual's physical and mental condition, the available treatments, and additional factors. A prognosis includes the expected duration, function, and description of the course of the disease, such as progressive decline, intermittent crisis, or sudden, unpredictable crisis.

The term "prediction" as used herein encompasses the probability of a disease and instituting preventive measures in order to either prevent the disease altogether or significantly decrease its impact upon the patient, such as by preventing mortality or limiting morbidity. The term further refers to clinical observations, it is to make an educated estimate about the natural history of a disease or its prognosis.

Furthermore, the term "predicting" as used herein means assessing whether a patient with COVID-19 as referred to in accordance with the method of the present invention will have a high disease severity, or not. In a particularly preferred embodiment, the disease severity is predicted.

In accordance with the present invention, the term "predicting the risk" is understood as an aid in the prediction of the risk of hospitalization due to an infection with COVID-19. The final prediction, in principle, will be carried out by physician and may include further diagnostic results.

The terms "significant" and "statistically significant" are known by the person skilled in the art. Thus, whether an increase or decrease is significant or statistically significant can be determined without further ado by the person skilled in the art using various well known statistic evaluation tools. For example, a significant increase or decrease is an increase or decrease of at least 10%, in particular of at least 20%.

The term "recommending" as used herein means establishing a proposal for a therapy which could be applied to the patient with COVID-19. However, it is to be understood that applying the actual therapy whatsoever is not comprised by the term. The therapy to be recommended depends on the outcome of the provided by the method of the present invention.

### Embodiments

In a first aspect, the present invention relates to a method for predicting the disease severity in a patient with Betavirus infection, said method comprising
a) determining the level of GDF-15 in a sample from the patient with Betavirus infection,
b) comparing the level or concentration determined in step a) to a reference, and
c) predicting the disease severity in a patient with Betavirus infection.

In an embodiment, the present invention relates to a method for predicting the disease severity in a patient with COVID-19, said method comprising
a) determining the level of GDF-15 in a sample from the patient with COVID-19,
b) comparing the level or concentration determined in step a) to a reference, and
c) predicting the disease severity in a patient with COVID-19.

In embodiments, it is predicted whether a patient with COVID-19 will develop a high disease severity in the future. In embodiments, it is predicted whether a patient with COVID-19 will develop a high disease severity within in the next, 24 to 72 hours.

In a preferred embodiment of the aforementioned method, an increased level of GDF-15 is indicative for an increased disease severity of a patient with COVID-19.

In particular, the level of GDF-15 in the sample of the patient is indicative for an increased disease severity of a patient with COVID-19 if the level of GDF-15 in the sample of the patient is higher than the level of GDF-15 in a reference or reference sample. In particular, GDF-15 is detectable in higher levels in a fluid sample of the patient with severe disease progression than in the same fluid sample of individuals with no or mild disease progression.

In a particular embodiments, the level of GDF-15 is increased in comparison to a reference. Preferably, said reference is a predetermined value. Said predetermined value shall allow for predicting the disease severity, for risk stratification, for monitoring disease progression, predicting the risk in a patient for hospital admission, for prediction the need for intensive care, for selecting for admission or treatment at Intensive Care Unit, for predicting the risk for mortality, for predicting the risk for thrombosis and for pulmonary embolism. It is to be understood that the reference may differ based on the type of prediction. E.g., the reference for the one or more GDF-15-type peptides for predicting the risk for mortality will be usually higher than the reference at the start point of the infection of a patient with COVID-19. However, this will be taken into account by the skilled person.

In embodiments, the sample from the patient is body fluid sample. In particular embodiments, the sample is a whole blood, serum or plasma sample. In embodiments, the sample is an in vitro sample, i.e. it will be analyzed in vitro and not transferred back into the body.

In particular embodiments, the patient is a laboratory animal, a domestic animal or a primate. In particular embodiments, the patient is a human patient.

In embodiments, the patient is a human patient. In embodiments, the patient is of any age. In embodiments, the patient is 50 years of age or older, in particular 60 years of age or older, and in particular 65 years of age or older. Further, it is envisaged that the patient to be tested is 70 years of age or older. Moreover, it is envisaged that the patient with COVID-19 to be tested is 75 years of age or older. Also, in embodiments the patient with COVID-19 may be between 50 and 90 years, in particular between 60 and 90 years, in particular between 70 and 90 years.

In embodiments, the level of GDF-15 is determined using antibodies, in particular using monoclonal antibodies. In embodiments, step a) of determining the level of GDF-15 in a sample of the patient comprises performing an immunoassay. In embodiments, the immunoassay is performed either in a direct or indirect format. In embodiments such immunoassays is selected from the group consisting of enzyme linked immunosorbent assay (ELISA), enzyme immunoassay (EIA), radioimmunoassay (RIA), or immuno assays based on detection of luminescence, fluorescence, chemiluminescence or electrochemiluminescence.

In particular embodiments, step a) of determining the level of GDF-15 in a sample of the patient comprises the steps of
i) incubating the sample of the patient with one or more antibodies specifically binding to GDF-15, thereby generating a complex between the antibody and GDF-15, and
ii) quantifying the complex formed in step i), thereby quantifying the level of GDF-15 in the sample of the patient.

In particular embodiments, in step i) the sample is incubated with two antibodies, specifically binding to GDF-15. As obvious to the skilled artisan, the sample can be contacted with the first and the second antibody in any desired order, i.e. first antibody first and then the second antibody or second antibody first and then the first antibody, or simultaneously, for a time and under conditions sufficient to form a first anti- GDF-15 antibody/ GDF-15 /second anti- GDF-15 antibody complex. As the skilled artisan will readily appreciate it is nothing but routine experimentation to establish the time and conditions that are appropriate or that are sufficient for the formation of a complex either between the specific anti- GDF-15 antibody and the GDF-15 antigen/analyte (= anti- GDF-15 complex) or the formation of the secondary, or sandwich complex comprising the first antibody to GDF-15, GDF-15 (the analyte) and the second anti - GDF-15 antibody (=anti-GDF-15 antibody/ GDF-15 /second anti- GDF-15 antibody complex).

The detection of the anti-GDF-15 antibody/GDF-15 complex can be performed by any appropriate means. The detection of the first anti-GDF-15 antibody/GDF-15/second anti-GDF-15 antibody complex can be performed by any appropriate means. The person skilled in the art is absolutely familiar with such means/methods.

In certain embodiments a sandwich will be formed comprising a first antibody to GDF-15, GDF-15 (analyte) and the second antibody to GDF-15, wherein the second antibody is detectably labeled.

In one embodiment a sandwich will be formed comprising a first antibody to GDF-15, the GDF-15 (analyte) and the second antibody to GDF-15, wherein the second antibody is detectably labeled and wherein the first anti- GDF-15 antibody is capable of binding to a solid phase or is bound to a solid phase.

In embodiments, the second antibody is directly or indirectly detectably labeled. In particular embodiments, the second antibody is detectably labeled with a luminescent dye, in particular a chemiluminescent dye or an electrochemiluminescent dye.

In a particular embodiment, an antigen in the sample, a biotinylated monoclonal GDF-15-specific antibody and a monoclonal GDF-15-specific antibody labeled with a ruthenium complex form a sandwich complex. After addition of streptavidin-coated microparticles, the complex becomes bound to the solid phase via interaction of biotin and streptavidin.

In a second aspect, the present invention relates to a method for risk stratification of a patient with Betavirus infection, said method comprising
a) determining the level of GDF-15 in a sample from the patient with Betavirus infection,
b) comparing the level determined in step a) to a reference,
c) stratifying the risk of a patient with Betavirus infection.

In an embodiment, the present invention relates to a method for risk stratification of a patient with COVID-19, said method comprising
a) determining the level of GDF-15 in a sample from the patient with COVID-19,
b) comparing the level determined in step a) to a reference,
c) ratifying the risk of a patient with COVID-19.

In a preferred embodiment of the aforementioned method, patients with an increase level of GDF-15 are stratified as having an increased risk for a more sever disease progression. Further, it is envisaged that the risk of a patient with COVID-19 is stratified.

In particular, the level of GDF-15 in the sample of the patient is indicative for an increased disease severity of a patient with COVID-19 if the level of GDF-15 in the sample of the patient is higher than the level of GDF-15 in a reference or reference sample. In particular, GDF-15 is detectable in higher levels in a fluid sample of the patient with severe disease progression than in the same fluid sample of individuals with no or mild disease progression.

In embodiments, risk stratification comprises categorization of COVID-19 patients to a particular risk status based on the level of GDF-15 measured in the sample of a COVID-19 patient. Thus, an increased level of GDF-15 of a patient with COVID-19 may aid the physician to a specific treatment or admission to the ICU.

Furthermore, the assessment of the level of GDF-15 in combination with clinical data such as clinical characteristics, medical history, detailed COVID-19 presentation, treatments, complications and outcomes may aid in guidance of the COVID-19 disease for stratifying the risk of a patient with COVID-19.

In embodiments, the sample from the patient is body fluid sample. In particular embodiments, the sample is a whole blood, serum or plasma sample. In embodiments, the sample is an in vitro sample, i.e. it will be analyzed in vitro and not transferred back into the body.

In particular embodiments, the patient is a laboratory animal, a domestic animal or a primate. In particular embodiments, the patient is a human patient.

In embodiments, the patient is a human patient. In embodiments, the patient is of any age. In embodiments, the patient is 50 years of age or older, in particular 60 years of age or older, and in particular 65 years of age or older. Further, it is envisaged that the patient to be tested is 70 years of age or older. Moreover, it is envisaged that the patient with COVID-19 to be tested is 75 years of age or older. Also, in embodiments the patient with COVID-19 may be between 50 and 90 years, in particular between 60 and 90 years, in particular between 70 and 90 years.

In embodiments, the level of GDF-15 is determined using antibodies, in particular using monoclonal antibodies. In embodiments, step a) of determining the level of GDF-15 in a sample of the patient comprises performing an immunoassay. In embodiments, the immunoassay is performed either in a direct or indirect format. In embodiments such immunoassays is selected from the group consisting of enzyme linked immunosorbent assay (ELISA), enzyme immunoassay (EIA), radioimmunoassay (RIA), or immuno assays based on detection of luminescence, fluorescence, chemiluminescence or electrochemiluminescence.

In particular embodiments, step a) of determining the level of GDF-15 in a sample of the patient comprises the steps of
i) incubating the sample of the patient with one or more antibodies specifically binding to GDF-15, thereby generating a complex between the antibody and GDF-15, and
ii) quantifying the complex formed in step i), thereby quantifying the level of GDF-15 in the sample of the patient.

In particular embodiments, in step i) the sample is incubated with two antibodies, specifically binding to GDF-15. As obvious to the skilled artisan, the sample can be contacted with the first and the second antibody in any desired order, i.e. first antibody first and then the second antibody or second antibody first and then the first antibody, or simultaneously, for a time and under conditions sufficient to form a first anti- GDF-15 antibody/ GDF-15 /second anti- GDF-15 antibody complex. As the skilled artisan will readily appreciate it is nothing but routine experimentation to establish the time and conditions that are appropriate or that are sufficient for the formation of a complex either between the specific anti- GDF-15 antibody and the GDF-15 antigen/analyte (= anti- GDF-15 complex) or the formation of the secondary, or sandwich complex comprising the first antibody to GDF-15, GDF-15 (the analyte) and the second anti - GDF-15 antibody (=anti-GDF-15 antibody/ GDF-15 /second anti- GDF-15 antibody complex).

The detection of the anti-GDF-15 antibody/GDF-15 complex can be performed by any appropriate means. The detection of the first anti-GDF-15 antibody/GDF-15/second anti-GDF-15 antibody complex can be performed by any appropriate means. The person skilled in the art is absolutely familiar with such means/methods.

In certain embodiments a sandwich will be formed comprising a first antibody to GDF-15, GDF-15 (analyte) and the second antibody to GDF-15, wherein the second antibody is detectably labeled.

In one embodiment a sandwich will be formed comprising a first antibody to GDF-15, the GDF-15 (analyte) and the second antibody to GDF-15, wherein the second antibody is detectably labeled and wherein the first anti- GDF-15 antibody is capable of binding to a solid phase or is bound to a solid phase.

In embodiments, the second antibody is directly or indirectly detectablly labeled. In particular embodiments, the second antibody is detectably labeled with a luminescent dye, in particular a chemiluminescent dye or an electrochemiluminescent dye.

In a particular embodiment, an antigen in the sample, a biotinylated monoclonal GDF-15-specific antibody and a monoclonal GDF-15-specific antibody labeled with a ruthenium complex form a sandwich complex. After addition of streptavidin-coated microparticles, the complex becomes bound to the solid phase via interaction of biotin and streptavidin.

In a third aspect, the present invention relates to a method for monitoring disease progression in a patient with Betavirus infection, said method comprising
a) determining the level of GDF-15 in a first sample from the patient with Betavirus infection,
b) determining the level of GDF-15 in a second sample from the patient with Betavirus infection which has been obtained after the first sample, and
c) comparing the level of GDF-15 in the first sample to the level of GDF-15 in the second sample, and
d) monitoring disease progression in a patient with Betavirus infection, based on the results of step c).

In an embodiment, the present invention relates to a method for monitoring disease progression in a patient with COVID-19, said method comprising
a) determining the level of GDF-15 in a first sample from the patient with COVID-19,
b) determining the level of GDF-15 in a second sample from the patient with COVID-19 which has been obtained after the first sample, and
c) comparing the level of GDF-15 in the first sample to the level of GDF-15 in the second sample, and
d) monitoring disease progression in a patient with COVID-19, based on the results of step c).

The aforementioned method may comprise the further step of monitoring the disease progression based on the results of the comparison step carried out in step c). As will be understood by those skilled in the art, the prediction of the disease severity in a patient with COVID-19 is usually not intended to be correct for 100% of the patient with COVID-19s. The term, however, requires that prediction can be made for a statistically significant portion of a patient with COVID-19 in a proper and correct manner. Thus, the actual monitoring may comprise further steps such as the confirmation.

In a preferred embodiment of the aforementioned method, the first sample is obtained after the determination of a COVID-19 infection or as early as first symptoms of a Cofic-19 infection.

In a further preferred embodiment of the aforementioned method the first sample from the patient with COVID-19 is obtained at admission into hospital.

In a preferred embodiment of the aforementioned method the second sample from the patient with COVID-19 is obtained at day 3 after admission into hospital.

In a preferred embodiment of the aforementioned method the second sample is taken at a later time point than the first sample.

Thus, the second sample shall be obtained after the first sample. It is to be understood that the second sample shall be obtained along the course of the COVID-19 infection of a patient.

Next to predicting the disease severity, it is also contemplated that the second sample is obtained to monitor the disease progression to see worsening or improvement of the disease before, during and after initiation of a therapy.

Moreover, it is particularly contemplated that the second sample is obtained after a reasonable period of time after obtaining the first sample. It is to be understood, that the levels of GDF-15 referred herein, do not instantly change (e.g. within 1 minute or 1 hour). Therefore, "reasonable" in this context refers to intervals between obtaining the first and second sample which intervals allow the biomarker to adjust.

Therefore, the second sample, preferably, is obtained at least 3 hours after said first sample, at least 23 hours, 72 hours, and 3 days up to 2 weeks after said first sample.

In a further preferred embodiment of the aforementioned method the time interval between taking the first and the second sample is at least 1 day, 2 days, 3 days, or 10 days.

In a further preferred embodiment of the aforementioned method wherein additional samples (third, fourth, fifth sample) are taken at a later time point.

In a preferred embodiment of the aforementioned method the third sample is taken at a later time point than the second sample.

In a further preferred embodiment of the aforementioned method the time interval between taking the second and the third sample is at least 3 hours, 1day, 3 days or 10 days.

In a preferred embodiment of the aforementioned method the fourth sample is taken at a later time point than the third sample.

In a further preferred embodiment of the aforementioned method the time interval between taking the third and the fourth sample is at least 3 hours, 1day, 3 days or 10 days.

In a further preferred embodiment of the aforementioned method wherein additional samples (fifth sample) are taken at a later time point. In a further preferred embodiment of the aforementioned method the time interval between taking the fourth and the fifth sample is at least 3 hours, 1day, 3 days or 10 days.

In a preferred embodiment of the aforementioned method the third sample from the patient with COVID-19 is obtained at day 2 after admission into hospital.

In a further preferred embodiment of the aforementioned method a sample from the patient with COVID-19 send to ICU is obtained daily.

In embodiments, the sample from the patient is body fluid sample. In particular embodiments, the sample is a whole blood, serum or plasma sample. In embodiments, the sample is an in vitro sample, i.e. it will be analyzed in vitro and not transferred back into the body.

In particular embodiments, the patient is a laboratory animal, a domestic animal or a primate. In particular embodiments, the patient is a human patient.

Preferably, by carrying out the method of the present invention it can be assessed whether the disease progression_of a patient with COVID-19 is more severe.

In a preferred embodiment of the aforementioned method an increased level of GDF-15 in the second sample is indicative of a more severe disease progression.

In particular, a level of GDF-15 in the second sample of the patient is indicative for a more severe disease progression in a patient with COVID-19 if the level of GDF-15 in the second sample of the patient is higher than the level of GDF-15 in the first sample. In particular, GDF-15 is detectable in higher levels in a second sample of the patient with severe disease progression than in the second sample of individuals with no or mild disease progression.

In a further preferred embodiment of the aforementioned method a patient with COVID-19 with an increased level of GDF-15 is monitored more closely.

In a preferred embodiment of the aforementioned an increased level of GDF-15 in the second sample is indicative that a patient with COVID-19 does not respond to a therapy.

Furthermore, by carrying out the method of the present invention it can be assessed whether the health status of a patient with COVID-19 is constant or improving.

Preferably, a decrease and, more preferably, a significant decrease, and, most preferably, a statistically significant decrease of the level of GDF-15 in the second sample as compared to the level of GDF-15 in the first sample is indicative for a patient with COVID-19 whose disease severity improves or who responds to the therapy for COVID-19. Thus, the therapy is efficient.

Also preferably, no change of the level of GDF-15 or an increase, more preferably, a significant increase, most preferably, a statistically significant increase of the level of GDF-15 in the second sample as compared to the level of GDF-15 in the first sample is indicative for a patient with COVID-19 that disease severity worsens or that a patient with COVID-19 does not respond to the therapy. Thus, the therapy is not efficient.

Furthermore, by carrying out the method of the present invention the disease progression can be predicted upon discharge of the patient from the hospital.

In embodiments, the patient is a human patient. In embodiments, the patient is of any age. In embodiments, the patient is 50 years of age or older, in particular 60 years of age or older, and in particular 65 years of age or older. Further, it is envisaged that the patient to be tested is 70 years of age or older. Moreover, it is envisaged that the patient with COVID-19 to be tested is 75 years of age or older. Also, in embodiments the patient with COVID-19 may be between 50 and 90 years, in particular between 60 and 90 years, in particular between 70 and 90 years.

In embodiments, the level of GDF-15 is determined using antibodies, in particular using monoclonal antibodies. In embodiments, steps a) and b) of determining the level of GDF-15 in a first and second sample of the patient comprises performing an immunoassay. In embodiments, the immunoassay is performed either in a direct or indirect format. In embodiments such immunoassays is selected from the group consisting of enzyme linked immunosorbent assay (ELISA), enzyme immunoassay (EIA), radioimmunoassay (RIA), or immuno assays based on detection of luminescence, fluorescence, chemiluminescence or electrochemiluminescence.

In particular embodiments, steps a) and b) of determining the level of GDF-15 in a first and second sample of the patient each individually comprises the steps of
i) incubating the sample of the patient with one or more antibodies specifically binding to GDF-15, thereby generating a complex between the antibody and GDF-15, and
ii) quantifying the complex formed in step i), thereby quantifying the level of GDF-15 in the sample of the patient.

In particular embodiments, in step i) the sample is incubated with two antibodies, specifically binding to GDF-15. As obvious to the skilled artisan, the sample can be contacted with the first and the second antibody in any desired order, i.e. first antibody first and then the second antibody or second antibody first and then the first antibody, or simultaneously, for a time and under conditions sufficient to form a first anti- GDF-15 antibody/ GDF-15 /second anti- GDF-15 antibody complex. As the skilled artisan will readily appreciate it is nothing but routine experimentation to establish the time and conditions that are appropriate or that are sufficient for the formation of a complex either between the specific anti- GDF-15 antibody and the GDF-15 antigen/analyte (= anti- GDF-15 complex) or the formation of the secondary, or sandwich complex comprising the first antibody to GDF-15, GDF-15 (the analyte) and the second anti - GDF-15 antibody (=anti-GDF-15 antibody/ GDF-15 /second anti- GDF-15 antibody complex).

The detection of the anti-GDF-15 antibody/GDF-15 complex can be performed by any appropriate means. The detection of the first anti-GDF-15 antibody/GDF-15/second anti-GDF-15 antibody complex can be performed by any appropriate means. The person skilled in the art is absolutely familiar with such means/methods.

In certain embodiments a sandwich will be formed comprising a first antibody to GDF-15, GDF-15 (analyte) and the second antibody to GDF-15, wherein the second antibody is detectably labeled.

In one embodiment a sandwich will be formed comprising a first antibody to GDF-15, the GDF-15 (analyte) and the second antibody to GDF-15, wherein the second antibody is detectably labeled and wherein the first anti- GDF-15 antibody is capable of binding to a solid phase or is bound to a solid phase.

In embodiments, the second antibody is directly or indirectly detectablly labeled. In particular embodiments, the second antibody is detectably labeled with a luminescent dye, in particular a chemiluminescent dye or an electrochemiluminescent dye.

In a particular embodiment, an antigen in a first sample, a biotinylated monoclonal GDF-15-specific antibody and a monoclonal GDF-15-specific antibody labeled with a ruthenium complex form a sandwich complex. After addition of streptavidin-coated microparticles, the complex becomes bound to the solid phase via interaction of biotin and streptavidin.

In a particular embodiment, an antigen in a second sample, a biotinylated monoclonal GDF-15-specific antibody and a monoclonal GDF-15-specific antibody labeled with a ruthenium complex form a sandwich complex. After addition of streptavidin-coated microparticles, the complex becomes bound to the solid phase via interaction of biotin and streptavidin.

The level of GDF-15 in the first sample to the level of GDF-15 in the second sample is afterwards compared.

Advantageously, it has been found in the studies underlying the present invention that the level of GDF-15 in a sample of a patient with COVID-19 allows for identifying patient with COVID-19s who are at risk of hospital admission.

Accordingly, in a fourth aspect, the present invention relates to a method of predicting the risk in a patient with Betavirus infection for hospital admission, said method comprising
b) determining the level of GDF-15 in a sample from the patient with Betavirus infection,
b) comparing the level determined in step a) to a reference,
c) predicting the risk for hospital admission in a patient with Betavirus infection.

In an embodiment, the present invention relates to a method of predicting the risk in a patient with COVID-19 for hospital admission, said method comprising
c) determining the level of GDF-15 in a sample from the patient with COVID-19,
b) comparing the level determined in step a) to a reference,
c) predicting the risk for hospital admission in a patient with COVID-19.

In a further preferred embodiment of the aforementioned method, hospital admission is recommended for a patient with an increased GDF-15 level. Thus, a patients with COVID-19 with none significant level of COVID-19 with mild and moderate illness may not require emergency interventions or hospitalization.

It is known that a SARS-CoV-2 infection can cause serious illness in some infected patients due to difficulty in breathing and which require hospital care. Around 1 in every 5 people who are infected with SARS-CoV-2, develop severe COVID-19. These patients at risk are typically, aged over 60 years, and people who have underlying medical conditions such as diabetes, heart disease, respiratory disease or hypertension are among those who are at greater risk. However, it has been described that also younger patients without such clinical risk factors may develop severe disease progression. Therefore, it is important to identify these high risk patient with COVID-19 as early as possible since this would allow for therapeutic measures that prevent or delay the progression.

In embodiments, the patient is admitted to a hospital, in particular, the patient is hospitalized due to an infection of the patient with SARS-CoV-2.

In accordance with the aforementioned method, the risk of a patient with COVID-19 for hospital admission due to an infection with SARS-CoV-2 shall be predicted. Thus, a patient with COVID-19 can be identified who is at risk for hospital admission due to an increased GDF-15 level, or who is not at risk for hospital admission.

As will be understood by those skilled in the art, the prediction of a risk is usually not intended to be correct for 100% of the patient with COVID-19. The term, preferably, means that the prediction can be made for a statistically significant portion of patient with COVID-19s in a proper and correct manner. Whether a portion is statistically significant can be determined without further ado by the person skilled in the art using various well known statistic evaluation tools, e.g., determination of confidence intervals, p-value determination, Student's t-test, Mann-Whitney test etc. Details are found in Dowdy and Wearden, Statistics for Research, John Wiley & Sons, New York 1983. Preferred confidence intervals are at least 90%, at least 95%, at least 97%, at least 98%, or at least 99%. The p-values are, preferably, 0.1, 0.05, 0.01, 0.005, or 0.0001.

Preferably, the risk/probability within a certain time window is predicted. In some embodiments, said predictive window is calculated from the completion of the method of the present invention. In particular, said predictive window is calculated from the time point at which the sample to be tested has been obtained.

In a preferred embodiment of the present invention, the predictive window, preferably, is an interval of at least 1 day, 2 days, at least 3 days, at least 4 days, at least 5 days, or at least 10 days, or any intermitting time range.

In another preferred embodiment of the present invention, the predictive window, preferably, is a period of up to , more preferably of up to 14 days, or most preferably, of up 21 days.

Thus, the risk within a period of up to three, up to five or up to nine days is predicted. Also, it is envisaged that the predictive window a period of 1 to 9 days. Alternatively, the predictive window may be a period of 1 to 3 days.

Preferably, the patient with COVID-19 to be analyzed by the above method of the present invention is allocated either into the group of patient with COVID-19 being at risk of hospital admission due to a COVID-19 infection, or into the group of patient with COVID-19 being not at risk of hospitalization due to a COVID-19 infection. A patient who is at risk, preferably, is a patient who has at elevated risk of hospital admission due to with COVID-19 infection (in particular within the predictive window). Preferably, said risk is elevated as compared to the risk in a cohort of patient with COVID-19 (i.e. a group of patients with COVID-19s). At patient who is not at risk, preferably, is a patient who has a reduced risk of hospital admission (in particular within the predictive window). Preferably, said risk is reduced as compared to the average risk in a cohort of patient with COVID-19 (i.e. a group of patients with COVID-19).

Accordingly, the method of the present invention allows for differentiating between an elevated risk and a reduced risk. A patient with COVID-19 who is at risk of preferably has a risk of 12% or larger, or, more preferably of 15% or larger, or most preferably of 20% or larger of hospital admission due to a COVID-19 infection, preferably, within a predictive window of 2 weeks

A patient with COVID-19 who is not at risk preferably has a risk of lower than 10%, more preferably of lower than, 8%, or most preferably of lower than 7% of hospitalization due to heart failure, preferably, within a predictive window of 2 weeks.

The term "reference" has been defined elsewhere herein. The definition applies accordingly. The reference to be applied in the above method shall allow for predicting the risk of hospitalization due to a COVID-19 infection. In some embodiments, the reference shall allow for differentiating between a patient with COVID-19 who is at risk of hospital admission and a patient with COVID-19 who is not at risk of hospitalization. In some embodiments, said reference is a predetermined value.

Preferably, the level of GDF-15 in the sample from a patient with COVID-19 which is increased as compared to the reference is indicative for a patient with COVID-19 being at risk of hospital admission. Also preferably, the level of GDF-15 in the sample from a patient with COVID-19 which is decreased as compared to the reference is indicative for a patient with COVID-19 who is not at risk of hospital admission.

In embodiments, the sample from the patient is body fluid sample. In particular embodiments, the sample is a whole blood, serum or plasma sample. In embodiments, the sample is an in vitro sample, i.e. it will be analyzed in vitro and not transferred back into the body.

In particular embodiments, the patient is a laboratory animal, a domestic animal or a primate. In particular embodiments, the patient is a human patient.

In embodiments, the patient is a human patient. In embodiments, the patient is of any age. In embodiments, the patient is 50 years of age or older, in particular 60 years of age or older, and in particular 65 years of age or older. Further, it is envisaged that the patient to be tested is 70 years of age or older. Moreover, it is envisaged that the patient with COVID-19 to be tested is 75 years of age or older. Also, in embodiments the patient with COVID-19 may be between 50 and 90 years, in particular between 60 and 90 years, in particular between 70 and 90 years.

In embodiments, the level of GDF-15 is determined using antibodies, in particular using monoclonal antibodies. In embodiments, step a) of determining the level of GDF-15 in a sample of the patient comprises performing an immunoassay. In embodiments, the immunoassay is performed either in a direct or indirect format. In embodiments such immunoassays is selected from the group consisting of enzyme linked immunosorbent assay (ELISA), enzyme immunoassay (EIA), radioimmunoassay (RIA), or immuno assays based on detection of luminescence, fluorescence, chemiluminescence or electrochemiluminescence.

In particular embodiments, step a) of determining the level of GDF-15 in a sample of the patient comprises the steps of
i) incubating the sample of the patient with one or more antibodies specifically binding to GDF-15, thereby generating a complex between the antibody and GDF-15, and
ii) quantifying the complex formed in step i), thereby quantifying the level of GDF-15 in the sample of the patient.

In particular embodiments, in step i) the sample is incubated with two antibodies, specifically binding to GDF-15. As obvious to the skilled artisan, the sample can be contacted with the first and the second antibody in any desired order, i.e. first antibody first and then the second antibody or second antibody first and then the first antibody, or simultaneously, for a time and under conditions sufficient to form a first anti- GDF-15 antibody/ GDF-15 /second anti- GDF-15 antibody complex. As the skilled artisan will readily appreciate it is nothing but routine experimentation to establish the time and conditions that are appropriate or that are sufficient for the formation of a complex either between the specific anti- GDF-15 antibody and the GDF-15 antigen/analyte (= anti- GDF-15 complex) or the formation of the secondary, or sandwich complex comprising the first antibody to GDF-15, GDF-15 (the analyte) and the second anti - GDF-15 antibody (=anti-GDF-15 antibody/ GDF-15 /second anti- GDF-15 antibody complex).

The detection of the anti-GDF-15 antibody/GDF-15 complex can be performed by any appropriate means. The detection of the first anti-GDF-15 antibody/GDF-15/second anti-GDF-15 antibody complex can be performed by any appropriate means. The person skilled in the art is absolutely familiar with such means/methods.

In certain embodiments a sandwich will be formed comprising a first antibody to GDF-15, GDF-15 (analyte) and the second antibody to GDF-15, wherein the second antibody is detectably labeled.

In one embodiment a sandwich will be formed comprising a first antibody to GDF-15, the GDF-15 (analyte) and the second antibody to GDF-15, wherein the second antibody is detectably labeled and wherein the first anti- GDF-15 antibody is capable of binding to a solid phase or is bound to a solid phase.

In embodiments, the second antibody is directly or indirectly detectablly labeled. In particular embodiments, the second antibody is detectably labeled with a luminescent dye, in particular a chemiluminescent dye or an electrochemiluminescent dye.

In a particular embodiment, an antigen in the sample, a biotinylated monoclonal GDF-15-specific antibody and a monoclonal GDF-15-specific antibody labeled with a ruthenium complex form a sandwich complex. After addition of streptavidin-coated microparticles, the complex becomes bound to the solid phase via interaction of biotin and streptavidin.

The method of the present invention may aid personalized medicine. In a preferred embodiment, the above method for predicting the risk of a patient with COVID-19 of hospital admission further comprises the step of recommending and/or initiating at least one suitable therapy, if it is predicted that the patient with COVID-19 is at risk of hospital admission. Accordingly, the present invention also pertains to a method of treatment.

In a fifth aspect, the present invention relates to a method for prediction the need for intensive care of a patient with Betavirus infection said method comprising
a) determining the level of GDF-15 in a sample from the patient with Betavirus infection
b) comparing the level determined in step a) to a reference,
c) predicting the need for intensive care of a patient with Betavirus infection.

In an embodiment, the present invention relates to a method for prediction the need for intensive care of a patient with COVID-19, said method comprising
a) determining the level of GDF-15 in a sample from the patient with COVID-19,
b) comparing the level determined in step a) to a reference,
c) predicting the need for intensive care of a patient with COVID-19.

In embodiments, it is predicted whether a patient with COVID-19 will need for intensive care in the future. In embodiments, it is predicted whether a patient with COVID-19 will need for intensive care within in the next, 24 to 72 hours.

In a preferred embodiment of the aforementioned method, an increased level of GDF-15 is indicative for the need for intensive care of a patient with COVID-19.

In particular, the level of GDF-15 in the sample of the patient is indicative for an increased the need for intensive care of a patient with COVID-19 if the level of GDF-15 in the sample of the patient is higher than the level of GDF-15 in a reference or reference sample. In particular, GDF-15 is detectable in higher levels in a fluid sample of the patient with severe disease progression than in the same fluid sample of individuals with no or mild disease progression.

In a particular embodiments, the level of GDF-15 is increased in comparison to a reference. Preferably, said reference is a predetermined value. Said predetermined value shall allow for predicting the disease severity, for risk stratification, for monitoring disease progression, predicting the risk in a patient for hospital admission, for prediction the need for intensive care, for selecting for admission or treatment at Intensive Care Unit, for predicting the risk for mortality, for predicting the risk for thrombosis and for pulmonary embolism. It is to be understood that the reference may differ based on the type of prediction. E.g., the reference for the one or more GDF-15-type peptides for predicting the risk for mortality will be usually higher than the reference at the startpoint of the infection of a patient with COVID-19. However, this will be taken into account by the skilled person.

In embodiments, the sample from the patient is body fluid sample. In particular embodiments, the sample is a whole blood, serum or plasma sample. In embodiments, the sample is an in vitro sample, i.e. it will be analyzed in vitro and not transferred back into the body.

In particular embodiments, the patient is a laboratory animal, a domestic animal or a primate. In particular embodiments, the patient is a human patient.

In embodiments, the patient is a human patient. In embodiments, the patient is of any age. In embodiments, the patient is 50 years of age or older, in particular 60 years of age or older, and in particular 65 years of age or older. Further, it is envisaged that the patient to be tested is 70 years of age or older. Moreover, it is envisaged that the patient with COVID-19 to be tested is 75 years of age or older. Also, in embodiments the patient with COVID-19 may be between 50 and 90 years, in particular between 60 and 90 years, in particular between 70 and 90 years.

In a further preferred embodiment of the aforementioned method, an increased level of GDF-15 is indicative for the need for intensive care of a patient with COVID-19. In particular, the level of GDF-15 in the sample of the patient is indicative for the need for intensive care of a patient with COVID-19 if the level of GDF-15 in the sample of the patient is higher than the level of GDF-15 in a reference or reference sample. In particular, GDF-15 is detectable in higher levels in a fluid sample of the patient in need of intensive care than in the same fluid sample of individuals with no need for intensive care.

In embodiments, the sample from the patient is body fluid sample. In particular embodiments, the sample is a whole blood, serum or plasma sample. In embodiments, the sample is an in vitro sample, i.e. it will be analyzed in vitro and not transferred back into the body.

In particular embodiments, the patient is a laboratory animal, a domestic animal or a primate. In particular embodiments, the patient is a human patient.

In embodiments, the patient is a human patient. In embodiments, the patient is of any age. In embodiments, the patient is 50 years of age or older, in particular 60 years of age or older, and in particular 65 years of age or older. Further, it is envisaged that the patient to be tested is 70 years of age or older. Moreover, it is envisaged that the patient with COVID-19 to be tested is 75 years of age or older. Also, in embodiments the patient with COVID-19 may be between 50 and 90 years, in particular between 60 and 90 years, in particular between 70 and 90 years.

In embodiments, the level of GDF-15 is determined using antibodies, in particular using monoclonal antibodies. In embodiments, step a) of determining the level of GDF-15 in a sample of the patient comprises performing an immunoassay. In embodiments, the immunoassay is performed either in a direct or indirect format. In embodiments such immunoassays is selected from the group consisting of enzyme linked immunosorbent assay (ELISA), enzyme immunoassay (EIA), radioimmunoassay (RIA), or immuno assays based on detection of luminescence, fluorescence, chemiluminescence or electrochemiluminescence.

In particular embodiments, step a) of determining the level of GDF-15 in a sample of the patient comprises the steps of
i) incubating the sample of the patient with one or more antibodies specifically binding to GDF-15, thereby generating a complex between the antibody and GDF-15, and
ii) quantifying the complex formed in step i), thereby quantifying the level of GDF-15 in the sample of the patient.

In particular embodiments, in step i) the sample is incubated with two antibodies, specifically binding to GDF-15. As obvious to the skilled artisan, the sample can be contacted with the first and the second antibody in any desired order, i.e. first antibody first and then the second antibody or second antibody first and then the first antibody, or simultaneously, for a time and under conditions sufficient to form a first anti- GDF-15 antibody/ GDF-15 /second anti- GDF-15 antibody complex. As the skilled artisan will readily appreciate it is nothing but routine experimentation to establish the time and conditions that are appropriate or that are sufficient for the formation of a complex either between the specific anti- GDF-15 antibody and the GDF-15 antigen/analyte (= anti- GDF-15 complex) or the formation of the secondary, or sandwich complex comprising the first antibody to GDF-15, GDF-15 (the analyte) and the second anti - GDF-15 antibody (=anti-GDF-15 antibody/ GDF-15 /second anti- GDF-15 antibody complex).

The detection of the anti-GDF-15 antibody/GDF-15 complex can be performed by any appropriate means. The detection of the first anti-GDF-15 antibody/GDF-15/second anti-GDF-15 antibody complex can be performed by any appropriate means. The person skilled in the art is absolutely familiar with such means/methods.

In certain embodiments a sandwich will be formed comprising a first antibody to GDF-15, GDF-15 (analyte) and the second antibody to GDF-15, wherein the second antibody is detectably labeled.

In one embodiment a sandwich will be formed comprising a first antibody to GDF-15, the GDF-15 (analyte) and the second antibody to GDF-15, wherein the second antibody is detectably labeled and wherein the first anti- GDF-15 antibody is capable of binding to a solid phase or is bound to a solid phase.

In embodiments, the second antibody is directly or indirectly detectablly labeled. In particular embodiments, the second antibody is detectably labeled with a luminescent dye, in particular a chemiluminescent dye or an electrochemiluminescent dye.

In a particular embodiment, an antigen in the sample, a biotinylated monoclonal GDF-15-specific antibody and a monoclonal GDF-15-specific antibody labeled with a ruthenium complex form a sandwich complex. After addition of streptavidin-coated microparticles, the complex becomes bound to the solid phase via interaction of biotin and streptavidin.

In a sixth aspect, the present invention relates to a method for selecting a patient with Betavirus infection for admission or treatment at Intensive Care Unit, said method comprising
a) determining the level of GDF-15 in a sample from the patient with Betavirus infection
b) comparing the level or concentration determined in step a) to a reference,
c) selecting a patient with Betavirus infection for an Intensive Care Unit.

In an embodiment, the present invention relates to a method for selecting a patient with COVID-19 for admission or treatment at Intensive Care Unit, said method comprising
a) determining the level of GDF-15 in a sample from the patient with COVID-19,
b) comparing the level or concentration determined in step a) to a reference,
c) selecting a patient with COVID-19 for an Intensive Care Unit.

In a further preferred embodiment of the aforementioned method, wherein patients with increased level of GDF-15 are selected for ICU admission or treatment.

In a further preferred embodiment of the aforementioned method, patients with COVID-19 selected for ICU are expected to have complications of their clinical outcome within 24 and 72 hours.

In a further preferred embodiment of the aforementioned method, a patient with a GDF-15 level in the fourth quartile of a COVID-19 population is classified as severe and/or critical and recommended for Intensive Care Unit. Preferably, the upper quartile of GDF-15 has been derived from a representative COVID-19 cohort and is preferentially at least 3000 pg/mL, 4000 pg/mL or 4500 pg/mL

In a further preferred embodiment of the aforementioned method the sample from the patient with COVID-19 is obtained to take in-hospital decisions for regular or intensive care unit (ICU).

In a further preferred embodiment of the aforementioned method the sample from the patient with COVID-19 is obtained at admission to the ICU. In a preferred embodiment of the aforementioned method the second sample from the patient with COVID-19 is obtained at admission to the ICU or at day 1 (24 hours), 2 (72 hours), 3 after admission to the ICU.

In a preferred embodiment of the aforementioned method the second sample is taken at a later time point than the first sample. Thus, the second sample shall be obtained after the first sample. It is to be understood that the second sample shall be obtained after the initiation the COVID-19 infection of a patient or after initiation of a therapy of a patient with COVID-19. However, it is also contemplated that the second sample may is obtained after worsening of the disease or after initiation of a therapy.

In a further preferred embodiment of the aforementioned method the third sample from the patient with COVID-19 is obtained at admission to the ICU or at day 1 (24 hours), 2 (72 hours), 3 after admission to the ICU.

It will be understood that an optimal sensitivity is desired for e.g. informing early about emerging severe complications including mortality within the next few days (24 hours, 72 hours) for a patient with COVID-19 already that has been selected for admission to the ICU at the time of admission to the ICU or 1, 2, 3 days after admission to the ICU.

In embodiments, the sample from the patient is body fluid sample. In particular embodiments, the sample is a whole blood, serum or plasma sample. In embodiments, the sample is an in vitro sample, i.e. it will be analyzed in vitro and not transferred back into the body.

In particular embodiments, the patient is a laboratory animal, a domestic animal or a primate. In particular embodiments, the patient is a human patient.

In embodiments, the patient is a human patient. In embodiments, the patient is of any age. In embodiments, the patient is 50 years of age or older, in particular 60 years of age or older, and in particular 65 years of age or older. Further, it is envisaged that the patient to be tested is 70 years of age or older. Moreover, it is envisaged that the patient with COVID-19 to be tested is 75 years of age or older. Also, in embodiments the patient with COVID-19 may be between 50 and 90 years, in particular between 60 and 90 years, in particular between 70 and 90 years.

In embodiments, the level of GDF-15 is determined using antibodies, in particular using monoclonal antibodies. In embodiments, step a) of determining the level of GDF-15 in a sample of the patient comprises performing an immunoassay. In embodiments, the immunoassay is performed either in a direct or indirect format. In embodiments such immunoassays is selected from the group consisting of enzyme linked immunosorbent assay (ELISA), enzyme immunoassay (EIA), radioimmunoassay (RIA), or immuno assays based on detection of luminescence, fluorescence, chemiluminescence or electrochemiluminescence.

In particular embodiments, step a) of determining the level of GDF-15 in a sample of the patient comprises the steps of
i) incubating the sample of the patient with one or more antibodies specifically binding to GDF-15, thereby generating a complex between the antibody and GDF-15, and
ii) quantifying the complex formed in step i), thereby quantifying the level of GDF-15 in the sample of the patient.

In particular embodiments, in step i) the sample is incubated with two antibodies, specifically binding to GDF-15. As obvious to the skilled artisan, the sample can be contacted with the first and the second antibody in any desired order, i.e. first antibody first and then the second antibody or second antibody first and then the first antibody, or simultaneously, for a time and under conditions sufficient to form a first anti- GDF-15 antibody/ GDF-15 /second anti- GDF-15 antibody complex. As the skilled artisan will readily appreciate it is nothing but routine experimentation to establish the time and conditions that are appropriate or that are sufficient for the formation of a complex either between the specific anti- GDF-15 antibody and the GDF-15 antigen/analyte (= anti- GDF-15 complex) or the formation of the secondary, or sandwich complex comprising the first antibody to GDF-15, GDF-15 (the analyte) and the second anti - GDF-15 antibody (=anti-GDF-15 antibody/ GDF-15 /second anti- GDF-15 antibody complex).

The detection of the anti-GDF-15 antibody/GDF-15 complex can be performed by any appropriate means. The detection of the first anti-GDF-15 antibody/GDF-15/second anti-GDF-15 antibody complex can be performed by any appropriate means. The person skilled in the art is absolutely familiar with such means/methods.

In certain embodiments a sandwich will be formed comprising a first antibody to GDF-15, GDF-15 (analyte) and the second antibody to GDF-15, wherein the second antibody is detectably labeled.

In one embodiment a sandwich will be formed comprising a first antibody to GDF-15, the GDF-15 (analyte) and the second antibody to GDF-15, wherein the second antibody is detectably labeled and wherein the first anti- GDF-15 antibody is capable of binding to a solid phase or is bound to a solid phase.

In embodiments, the second antibody is directly or indirectly detectably labeled. In particular embodiments, the second antibody is detectably labeled with a luminescent dye, in particular a chemiluminescent dye or an electrochemiluminescent dye.

In a particular embodiment, an antigen in the sample, a biotinylated monoclonal GDF-15-specific antibody and a monoclonal GDF-15-specific antibody labeled with a ruthenium complex form a sandwich complex. After addition of streptavidin-coated microparticles, the complex becomes bound to the solid phase via interaction of biotin and streptavidin.

In a seventh aspect, the present invention relates to a method of method for predicting the risk for mortality in a patient with Betavirus infection, said method comprising
a) determining the level of GDF-15 in a sample from the patient with Betavirus infection,
b) comparing the level determined in step a) to a reference,
c) predicting the risk for mortality in a patient with Betavirus infection.

In an embodiment, the present invention relates to a method of method for predicting the risk for mortality in a patient with COVID-19, said method comprising
a) determining the level of GDF-15 in a sample from the patient with COVID-19,
b) comparing the level determined in step a) to a reference,
c) predicting the risk for mortality in a patient with COVID-19.

In a further preferred embodiment of the aforementioned method, an increased level of GDF-15 is indicative for an increased risk for mortality in a patient with COVID-19. In particular, the level of GDF-15 in the sample of the patient is indicative for an increased risk for mortality of a patient with COVID-19 if the level of GDF-15 in the sample of the patient is higher than the level of GDF-15 in a reference or reference sample. In particular, GDF-15 is detectable in higher levels in a fluid sample of the patient with a higher risk of mortality than in the same fluid sample of individuals with a low risk of mortality.

In a further preferred embodiment of the aforementioned method, a GDF-15 level in the third and fourth quartiles is associated with a higher risk of mortality.

In embodiments, the sample from the patient is body fluid sample. In particular embodiments, the sample is a whole blood, serum or plasma sample. In embodiments, the sample is an in vitro sample, i.e. it will be analyzed in vitro and not transferred back into the body.

In particular embodiments, the patient is a laboratory animal, a domestic animal or a primate. In particular embodiments, the patient is a human patient.

In embodiments, the patient is a human patient. In embodiments, the patient is of any age. In embodiments, the patient is 50 years of age or older, in particular 60 years of age or older, and in particular 65 years of age or older. Further, it is envisaged that the patient to be tested is 70 years of age or older. Moreover, it is envisaged that the patient with COVID-19 to be tested is 75 years of age or older. Also, in embodiments the patient with COVID-19 may be between 50 and 90 years, in particular between 60 and 90 years, in particular between 70 and 90 years.

In embodiments, the level of GDF-15 is determined using antibodies, in particular using monoclonal antibodies. In embodiments, step a) of determining the level of GDF-15 in a sample of the patient comprises performing an immunoassay. In embodiments, the immunoassay is performed either in a direct or indirect format. In embodiments such immunoassays is selected from the group consisting of enzyme linked immunosorbent assay (ELISA), enzyme immunoassay (EIA), radioimmunoassay (RIA), or immuno assays based on detection of luminescence, fluorescence, chemiluminescence or electrochemiluminescence.

In particular embodiments, step a) of determining the level of GDF-15 in a sample of the patient comprises the steps of
i) incubating the sample of the patient with one or more antibodies specifically binding to GDF-15, thereby generating a complex between the antibody and GDF-15, and
ii) quantifying the complex formed in step i), thereby quantifying the level of GDF-15 in the sample of the patient.

In particular embodiments, in step i) the sample is incubated with two antibodies, specifically binding to GDF-15. As obvious to the skilled artisan, the sample can be contacted with the first and the second antibody in any desired order, i.e. first antibody first and then the second antibody or second antibody first and then the first antibody, or simultaneously, for a time and under conditions sufficient to form a first anti- GDF-15 antibody/ GDF-15 /second anti- GDF-15 antibody complex. As the skilled artisan will readily appreciate it is nothing but routine experimentation to establish the time and conditions that are appropriate or that are sufficient for the formation of a complex either between the specific anti- GDF-15 antibody and the GDF-15 antigen/analyte (= anti- GDF-15 complex) or the formation of the secondary, or sandwich complex comprising the first antibody to GDF-15, GDF-15 (the analyte) and the second anti - GDF-15 antibody (=anti-GDF-15 antibody/ GDF-15 /second anti- GDF-15 antibody complex).

The detection of the anti-GDF-15 antibody/GDF-15 complex can be performed by any appropriate means. The detection of the first anti-GDF-15 antibody/GDF-15/second anti-GDF-15 antibody complex can be performed by any appropriate means. The person skilled in the art is absolutely familiar with such means/methods.

In certain embodiments a sandwich will be formed comprising a first antibody to GDF-15, GDF-15 (analyte) and the second antibody to GDF-15, wherein the second antibody is detectably labeled.

In one embodiment a sandwich will be formed comprising a first antibody to GDF-15, the GDF-15 (analyte) and the second antibody to GDF-15, wherein the second antibody is detectably labeled and wherein the first anti- GDF-15 antibody is capable of binding to a solid phase or is bound to a solid phase.

In embodiments, the second antibody is directly or indirectly detectablly labeled. In particular embodiments, the second antibody is detectably labeled with a luminescent dye, in particular a chemiluminescent dye or an electrochemiluminescent dye.

In a particular embodiment, an antigen in the sample, a biotinylated monoclonal GDF-15-specific antibody and a monoclonal GDF-15-specific antibody labeled with a ruthenium complex form a sandwich complex. After addition of streptavidin-coated microparticles, the complex becomes bound to the solid phase via interaction of biotin and streptavidin.

In an eighth aspect, the present invention relates to an *in vitro* use of GDF-15 or of an agent which binds to GDF-15 for
a) predicting the disease severity of a patient with Betavirus infection,
b) risk stratification of a patient with Betavirus infection,
c) monitoring the extent of the disease severity of a patient with Betavirus infection.

In an embodiment, the present invention relates to an *in vitro* use of GDF-15 or of an agent which binds to GDF-15 for
a) predicting the disease severity of a patient with COVID-19,
b) risk stratification of a patient with COVID-19,
c) monitoring the extent of the disease severity of a patient with COVID-19.

The present invention concerns the use (in particular, the in vitro use, e.g. in a sample from a patient with COVID-19) of a GFD-15 peptide, and/or of at least one agent that specifically binds to a GDF-15 peptide for predicting the disease severity of a patient with COVID-19, for risk stratification of a patient with COVID-19, and monitoring the extent of the disease severity of a patient with COVID-19.

In embodiments, the sample from the patient is body fluid sample. In particular embodiments, the sample is a whole blood, serum or plasma sample. In embodiments, the sample is an in vitro sample, i.e. it will be analyzed in vitro and not transferred back into the body.

In particular embodiments, the patient is a laboratory animal, a domestic animal or a primate. In particular embodiments, the patient is a human patient.

In embodiments, the patient is a human patient. In embodiments, the patient is of any age. In embodiments, the patient is 50 years of age or older, in particular 60 years of age or older, and in particular 65 years of age or older. Further, it is envisaged that the patient to be tested is 70 years of age or older. Moreover, it is envisaged that the patient with COVID-19 to be tested is 75 years of age or older. Also, in embodiments the patient with COVID-19 may be between 50 and 90 years, in particular between 60 and 90 years, in particular between 70 and 90 years.

In embodiments, the level of GDF-15 is determined using antibodies, in particular using monoclonal antibodies. In embodiments, step a) of determining the level of GDF-15 in a sample of the patient comprises performing an immunoassay. In embodiments, the immunoassay is performed either in a direct or indirect format. In embodiments such immunoassays is selected from the group consisting of enzyme linked immunosorbent assay (ELISA), enzyme immunoassay (EIA), radioimmunoassay (RIA), or immuno assays based on detection of luminescence, fluorescence, chemiluminescence or electrochemiluminescence.

In particular embodiments, step a) of determining the level of GDF-15 in a sample of the patient comprises the steps of
i) incubating the sample of the patient with one or more antibodies specifically binding to GDF-15, thereby generating a complex between the antibody and GDF-15, and
ii) quantifying the complex formed in step i), thereby quantifying the level of GDF-15 in the sample of the patient.

In particular embodiments, in step i) the sample is incubated with two antibodies, specifically binding to GDF-15. As obvious to the skilled artisan, the sample can be contacted with the first and the second antibody in any desired order, i.e. first antibody first and then the second antibody or second antibody first and then the first antibody, or simultaneously, for a time and under conditions sufficient to form a first anti- GDF-15 antibody/ GDF-15 /second anti- GDF-15 antibody complex. As the skilled artisan will readily appreciate it is nothing but routine experimentation to establish the time and conditions that are appropriate or that are sufficient for the formation of a complex either between the specific anti- GDF-15 antibody and the GDF-15 antigen/analyte (= anti- GDF-15 complex) or the formation of the secondary, or sandwich complex comprising the first antibody to GDF-15, GDF-15 (the analyte) and the second anti - GDF-15 antibody (=anti-GDF-15 antibody/ GDF-15 /second anti- GDF-15 antibody complex).

The detection of the anti-GDF-15 antibody/GDF-15 complex can be performed by any appropriate means. The detection of the first anti-GDF-15 antibody/GDF-15/second anti-GDF-15 antibody complex can be performed by any appropriate means. The person skilled in the art is absolutely familiar with such means/methods.

In certain embodiments a sandwich will be formed comprising a first antibody to GDF-15, GDF-15 (analyte) and the second antibody to GDF-15, wherein the second antibody is detectably labeled.

In one embodiment a sandwich will be formed comprising a first antibody to GDF-15, the GDF-15 (analyte) and the second antibody to GDF-15, wherein the second antibody is detectably labeled and wherein the first anti- GDF-15 antibody is capable of binding to a solid phase or is bound to a solid phase.

In embodiments, the second antibody is directly or indirectly detectablly labeled. In particular embodiments, the second antibody is detectably labeled with a luminescent dye, in particular a chemiluminescent dye or an electrochemiluminescent dye.

In a particular embodiment, an antigen in the sample, a biotinylated monoclonal GDF-15-specific antibody and a monoclonal GDF-15-specific antibody labeled with a ruthenium complex form a sandwich complex. After addition of streptavidin-coated microparticles, the complex becomes bound to the solid phase via interaction of biotin and streptavidin.

In a ninth aspect, the present invention relates to a method for predicting the risk for thrombosis in a patient with Betavirus infection, said method comprising
a) determining the level of GDF-15 in a sample from the patient with Betavirus infection,
b) comparing the level determined in step a) to a reference,
c) predicting the risk for thrombosis of a patient with Betavirus infection.

In an embodiment, the present invention relates to a method for predicting the risk for thrombosis in a patient with COVID-19, said method comprising
a) determining the level of GDF-15 in a sample from the patient with COVID-19,
b) comparing the level determined in step a) to a reference,
c) predicting the risk for thrombosis of a patient with COVID-19.

In a further preferred embodiment of the aforementioned method, an increased level of GDF-15 is indicative for an increased risk of thrombosis in a patient with COVID-19. In particular, the level of GDF-15 in the sample of the patient is indicative for an increased risk for thrombosis of a patient with COVID-19 if the level of GDF-15 in the sample of the patient is higher than the level of GDF-15 in a reference or reference sample. In particular, GDF-15 is detectable in higher levels in a fluid sample of the patient with a higher risk for thrombosis than in the same fluid sample of individuals with a lower risk for thrombosis.

In embodiments, the sample from the patient is body fluid sample. In particular embodiments, the sample is a whole blood, serum or plasma sample. In embodiments, the sample is an in vitro sample, i.e. it will be analyzed in vitro and not transferred back into the body.

In particular embodiments, the patient is a laboratory animal, a domestic animal or a primate. In particular embodiments, the patient is a human patient.

In embodiments, the patient is a human patient. In embodiments, the patient is of any age. In embodiments, the patient is 50 years of age or older, in particular 60 years of age or older, and in particular 65 years of age or older. Further, it is envisaged that the patient to be tested is 70 years of age or older. Moreover, it is envisaged that the patient with COVID-19 to be tested is 75 years of age or older. Also, in embodiments the patient with COVID-19 may be between 50 and 90 years, in particular between 60 and 90 years, in particular between 70 and 90 years.

In embodiments, the level of GDF-15 is determined using antibodies, in particular using monoclonal antibodies. In embodiments, step a) of determining the level of GDF-15 in a sample of the patient comprises performing an immunoassay. In embodiments, the immunoassay is performed either in a direct or indirect format. In embodiments such immunoassays is selected from the group consisting of enzyme linked immunosorbent assay (ELISA), enzyme immunoassay (EIA), radioimmunoassay (RIA), or immuno assays based on detection of luminescence, fluorescence, chemiluminescence or electrochemiluminescence.

In particular embodiments, step a) of determining the level of GDF-15 in a sample of the patient comprises the steps of
i) incubating the sample of the patient with one or more antibodies specifically binding to GDF-15, thereby generating a complex between the antibody and GDF-15, and
ii) quantifying the complex formed in step i), thereby quantifying the level of GDF-15 in the sample of the patient.

In particular embodiments, in step i) the sample is incubated with two antibodies, specifically binding to GDF-15. As obvious to the skilled artisan, the sample can be contacted with the first and the second antibody in any desired order, i.e. first antibody first and then the second antibody or second antibody first and then the first antibody, or simultaneously, for a time and under conditions sufficient to form a first anti- GDF-15 antibody/ GDF-15 /second anti- GDF-15 antibody complex. As the skilled artisan will readily appreciate it is nothing but routine experimentation to establish the time and conditions that are appropriate or that are sufficient for the formation of a complex either between the specific anti- GDF-15 antibody and the GDF-15 antigen/analyte (= anti- GDF-15 complex) or the formation of the secondary, or sandwich complex comprising the first antibody to GDF-15, GDF-15 (the analyte) and the second anti - GDF-15 antibody (=anti-GDF-15 antibody/ GDF-15 /second anti- GDF-15 antibody complex).

The detection of the anti-GDF-15 antibody/GDF-15 complex can be performed by any appropriate means. The detection of the first anti-GDF-15 antibody/GDF-15/second anti-GDF-15 antibody complex can be performed by any appropriate means. The person skilled in the art is absolutely familiar with such means/methods.

In certain embodiments a sandwich will be formed comprising a first antibody to GDF-15, GDF-15 (analyte) and the second antibody to GDF-15, wherein the second antibody is detectably labeled.

In one embodiment a sandwich will be formed comprising a first antibody to GDF-15, the GDF-15 (analyte) and the second antibody to GDF-15, wherein the second antibody is detectably labeled and wherein the first anti- GDF-15 antibody is capable of binding to a solid phase or is bound to a solid phase.

In embodiments, the second antibody is directly or indirectly detectablly labeled. In particular embodiments, the second antibody is detectably labeled with a luminescent dye, in particular a chemiluminescent dye or an electrochemiluminescent dye.

In a particular embodiment, an antigen in the sample, a biotinylated monoclonal GDF-15-specific antibody and a monoclonal GDF-15-specific antibody labeled with a ruthenium complex form a sandwich complex. After addition of streptavidin-coated microparticles, the complex becomes bound to the solid phase via interaction of biotin and streptavidin.

In a preferred embodiment, the method for predicting the risk of thrombosis in a patient with COVID-19 further comprises i) the step of recommending anticoagulation therapy, or ii) of recommending an intensification of anticoagulation therapy, if the patient with COVID-19 has been identified to be at risk to suffer from thrombosis.

In a preferred embodiment, the method for predicting the risk of thrombosis in a patient with COVID-19 further comprises i) the step of initiating anticoagulation therapy, or ii) of intensifying anticoagulation therapy, if the patient with COVID-19 has been identified to be at risk to suffer from thrombosis (by the method of the present invention).

If the test patient with COVID-19 is on anticoagulation therapy, and if the patient with COVID-19 has been identified not to be at risk to suffer from thrombosis (by the method of the present invention) the dosage of anticoagulation therapy may be reduced. Accordingly, a reduction of the dosage may be recommended. Be reducing the dosage, the risk to suffer from side effects (such as bleeding) may be reduced.

In particular, the following applies:
If the patient with COVID-19 to be tested does not receive anticoagulation therapy, the initiation of anticoagulation is recommended, if the patient with COVID-19 has been identified to be at risk to suffer from stroke. Thus, anticoagulation therapy shall be initiated.
If the patient with COVID-19 to be tested already receives anticoagulation therapy, the intensification of anticoagulation is recommended, if the patient with COVID-19 has been identified to be at risk to suffer from thrombosis. Thus, anticoagulation therapy shall be intensified.

In a preferred embodiment, anticoagulation therapy is intensified by increasing the dosage of the anticoagulant, i.e. the dosage of the currently administered coagulant.

In a particularly preferred embodiment, anticoagulation therapy is intensified by increasing the replacing the currently administered anticoagulant with a more effective anticoagulant. Thus, a replacement of the anticoagulant is recommended.

It has been described that better prevention in high risk patients is achieved with the oral anticoagulant apixaban versus the vitamin K antagonist warfarin as shown in Hijazi at al., The Lancet 2016 387, 2302-2311,(Figure 4).

Thus, it is envisaged that the patient with COVID-19 to be tested is a patient who is treated with a vitamin K antagonist such as warfarin or dicumarol. If the patient with COVID-19 has been identified to be at risk to suffer from thrombosis (by the method of the present invention, it the replacement of the vitamin K antagonist with an oral anticoagulant, in particular dabigatran, rivaroxaban or apixaban is recommended. Accordingly, the therapy with the vitamin K antagonist is discontinued and therapy with an oral anticoagulant is initiated.

As used herein, the term "assessing a therapy for thrombosis", preferably refers to the assessment of a therapy that aims to treat COVID-19 complications such as venous and arterial thrombosis and pulmonary embolism and increased rates of thrombosis reported in a series of COVID-19 patients. Because anticoagulation is introducing a certain bleeding risk, the need of anticoagulation therapy shall be assessed.

In particular embodiments the assessment of thrombosis therapy includes the assessments of complications selected from the group consisting of venous thrombosis, arterial thrombosis, pulmonary embolism and increased rates of thrombosis.

The therapy to be assessed can be any therapy that aims to treat thrombosis. Preferably the therapy is the administration of at least one anticoagulant, i.e. anticoagulation therapy. Anticoagulation therapy is preferably a therapy which aims to reduce the risk of anticoagulation in said patient with COVID-19. Administration of at least one anticoagulant shall aim to reduce or prevent coagulation of blood and related stroke. In a preferred embodiment, at least one anticoagulant is selected from the group consisting of heparin, a coumarin derivative (i.e. a vitamin K antagonist), in particular warfarin or dicumarol, oral anticoagulants, in particular dabigatran, rivaroxaban or apixaban, tissue factor pathway inhibitor (TFPI), antithrombin III, factor IXa inhibitors, factor Xa inhibitors, inhibitors of factors Va and VIIIa and thrombin inhibitors (anti-IIa type). Accordingly, it is envisaged that the patient with COVID-19 takes at least one of the aforementioned medicaments.

In preferred embodiment, the anticoagulant is a vitamin K antagonist such as warfarin or dicumarol. Vitamin K antagonists, such as warfarin or dicumarol are less expensive, but need better patient compliance, because of the inconvenient, cumbersome and often unreliable treatment with fluctuating time in therapeutic range. NOAC (new oral anticoagulants) comprise direct factor Xa inhibitors (apixaban, rivaroxaban, darexaban, edoxaban), direct thrombin inhibitors (dabigatran) and PAR-1 antagonists (vorapaxar, atopaxar).

In another preferred embodiment the anticoagulant and oral anticoagulant, in particular apixaban, rivaroxaban, darexaban, edoxaban, dabigatran, vorapaxar, or atopaxar.

Thus, the patient with COVID-19 to be tested may be on therapy with an oral anticoagulant or a vitamin K antagonist at the time of the testing (i.e. at the time at which the sample is received).

In a tenth aspect, the present invention relates to a method for predicting the risk for pulmonary embolism of a patient with Betavirus infection, said method comprising
a) determining the level of GDF-15 in a sample from the patient with Betavirus infection,
b) comparing the level determined in step a) to a reference,
c) predicting the risk for pulmonary embolism of a patient with Betavirus infection.

In an embodiment, the present invention relates to a method for predicting the risk for pulmonary embolism of a patient with COVID-19, said method comprising
a) determining the level of GDF-15 in a sample from the patient with COVID-19,
b) comparing the level determined in step a) to a reference,
c) predicting the risk for pulmonary embolism of a patient with COVID-19.

In a further preferred embodiment of the aforementioned method, an increased level of GDF-15 is indicative for an increased risk for pulmonary embolism of a patient with COVID-19.

In a further preferred embodiment of the aforementioned method, an increased level of GDF-15 is indicative for the increased need of mechanical ventilation and prevention of hypoxia. In particular, the level of GDF-15 in the sample of the patient is indicative for the increased need of mechanical ventilation and prevention of hypoxia of a patient with COVID-19 if the level of GDF-15 in the sample of the patient is higher than the level of GDF-15 in a reference or reference sample. In particular, GDF-15 is detectable in higher levels in a fluid sample of the patient with increased need of mechanical ventilation and prevention of hypoxia than in the same fluid sample of individuals with no need of mechanical ventilation and prevention of hypoxia.

In embodiments, the sample from the patient is body fluid sample. In particular embodiments, the sample is a whole blood, serum or plasma sample. In embodiments, the sample is an in vitro sample, i.e. it will be analyzed in vitro and not transferred back into the body.

In particular embodiments, the patient is a laboratory animal, a domestic animal or a primate. In particular embodiments, the patient is a human patient.

In embodiments, the patient is a human patient. In embodiments, the patient is of any age. In embodiments, the patient is 50 years of age or older, in particular 60 years of age or older, and in particular 65 years of age or older. Further, it is envisaged that the patient to be tested is 70 years of age or older. Moreover, it is envisaged that the patient with COVID-19 to be tested is 75 years of age or older. Also, in embodiments the patient with COVID-19 may be between 50 and 90 years, in particular between 60 and 90 years, in particular between 70 and 90 years.

In embodiments, the level of GDF-15 is determined using antibodies, in particular using monoclonal antibodies. In embodiments, step a) of determining the level of GDF-15 in a sample of the patient comprises performing an immunoassay. In embodiments, the immunoassay is performed either in a direct or indirect format. In embodiments such immunoassays is selected from the group consisting of enzyme linked immunosorbent assay (ELISA), enzyme immunoassay (EIA), radioimmunoassay (RIA), or immuno assays based on detection of luminescence, fluorescence, chemiluminescence or electrochemiluminescence.

In particular embodiments, step a) of determining the level of GDF-15 in a sample of the patient comprises the steps of
i) incubating the sample of the patient with one or more antibodies specifically binding to GDF-15, thereby generating a complex between the antibody and GDF-15, and
ii) quantifying the complex formed in step i), thereby quantifying the level of GDF-15 in the sample of the patient.

In particular embodiments, in step i) the sample is incubated with two antibodies, specifically binding to GDF-15. As obvious to the skilled artisan, the sample can be contacted with the first and the second antibody in any desired order, i.e. first antibody first and then the second antibody or second antibody first and then the first antibody, or simultaneously, for a time and under conditions sufficient to form a first anti- GDF-15 antibody/ GDF-15 /second anti- GDF-15 antibody complex. As the skilled artisan will readily appreciate it is nothing but routine experimentation to establish the time and conditions that are appropriate or that are sufficient for the formation of a complex either between the specific anti- GDF-15 antibody and the GDF-15 antigen/analyte (= anti- GDF-15 complex) or the formation of the secondary, or sandwich complex comprising the first antibody to GDF-15, GDF-15 (the analyte) and the second anti - GDF-15 antibody (=anti-GDF-15 antibody/ GDF-15 /second anti- GDF-15 antibody complex).

The detection of the anti-GDF-15 antibody/GDF-15 complex can be performed by any appropriate means. The detection of the first anti-GDF-15 antibody/GDF-15/second anti-GDF-15 antibody complex can be performed by any appropriate means. The person skilled in the art is absolutely familiar with such means/methods.

In certain embodiments a sandwich will be formed comprising a first antibody to GDF-15, GDF-15 (analyte) and the second antibody to GDF-15, wherein the second antibody is detectably labeled.

In one embodiment a sandwich will be formed comprising a first antibody to GDF-15, the GDF-15 (analyte) and the second antibody to GDF-15, wherein the second antibody is detectably labeled and wherein the first anti- GDF-15 antibody is capable of binding to a solid phase or is bound to a solid phase.

In embodiments, the second antibody is directly or indirectly detectablly labeled. In particular embodiments, the second antibody is detectably labeled with a luminescent dye, in particular a chemiluminescent dye or an electrochemiluminescent dye.

In a particular embodiment, an antigen in the sample, a biotinylated monoclonal GDF-15-specific antibody and a monoclonal GDF-15-specific antibody labeled with a ruthenium complex form a sandwich complex. After addition of streptavidin-coated microparticles, the complex becomes bound to the solid phase via interaction of biotin and streptavidin.

In an eleventh aspect, the present invention relates to a method for a computer-implemented method for predicting the disease severity in a patient with Betavirus infection, said method comprising
a) receiving at a processing unit a value for the level of GDF-15 in a sample from a patient with Betavirus infection,
b) processing the value received in step (a) with the processing unit, wherein said processing comprises retrieving from a memory one or more threshold values for the level of GDF-15 and comparing the value received in step (a) with the one or more threshold values, and
c) providing a prediction of the disease severity of a patient with Betavirus infection via an output device, wherein said prediction is based on the results of step (b).

In an embodiment, the present invention relates to a method for a computer-implemented method for predicting the disease severity in a patient with COVID-19, said method comprising
a) receiving at a processing unit a value for the level of GDF-15 in a sample from a patient with COVID-19,
b) processing the value received in step (a) with the processing unit, wherein said processing comprises retrieving from a memory one or more threshold values for the level of GDF-15 and comparing the value received in step (a) with the one or more threshold values, and
c) providing a prediction of the disease severity of a patient with COVID-19 via an output device, wherein said prediction is based on the results of step (b).

The above-mentioned method is a computer-implemented method. Preferably, all steps of the computer-implemented method are performed by one or more processing units of a computer (or computer network). Thus, the assessment in step (c) is carried out by a processing unit. Preferably, said assessment is based on the results of step (b).

The value or values received in step (a) shall be derived from the determination of the level of the biomarker from a patient with COVID-19 as described elsewhere herein. Preferably, the value is a value for the concentration of the biomarker. The value will be typically received by the processing unit by uploading or sending the value to the processing unit. Alternatively, the value can be received by the processing unit by inputting the value via an user interface.

In an embodiment of the aforementioned method, the reference (or references) set forth in step (b) is (are) established from a memory. Preferably, a value for the reference is established from the memory.

In an embodiment of the aforementioned computer-implemented method of the present invention, the result of the assessment made in step c) is provided via a display, configured for presenting result.

In an embodiment of the aforementioned computer-implemented method of the present invention, the method may comprise the further step of transferring the information on the assessment made in step c) to the patient with COVID-19's electronic medical records.

In a twelfth aspect, the present invention relates to a method for an *in vitro* use of GDF-15 to diagnose Betavirus infection patients with a
a) decreased oxygen in blood (hypoxia),
b) silent hypoxia, where the patient and physicians might not have been aware of the hypoxia and disease severity based on clinical symptoms,
c) under perfusion (ischemia), insufficient oxygen supply and necrosis of tissue (generic hypoxia).

In an embodiment, the present invention relates to a method for an *in vitro* use of GDF-15 to diagnose COVID-19 patients with a
a) decreased oxygen in blood (hypoxia),
b) silent hypoxia, where the patient and physicians might not have been aware of the hypoxia and disease severity based on clinical symptoms,
c) under perfusion (ischemia), insufficient oxygen supply and necrosis of tissue (generic hypoxia).

In a preferred embodiment of the aforementioned method, hypoxia and insufficient tissue oxygenation is clinically not visible ("asymptomatic") in some patients with COVID-19, termed silent hypoxia.

Preferably, the aforementioned uses are an *in vitro* uses. Moreover, the detection agent is preferably and antibody such as a monoclonal antibody (or an antigen binding fragment thereof).

The present invention also relates to a kit. In an embodiment, the kit of the present invention comprises at least one agent which specifically binds to a GDF-15 peptide. Preferably, said kit is adapted for carrying out the method of the present invention, i.e. the method for predicting the disease severity, or a method for risk stratification or method for monitoring disease progression in a patient with COVID-19. Optionally, said kit comprises instructions for carrying out the said method.

In an embodiment, said kit is used for predicting the disease severity *in vitro.* In an alternative embodiment, said kit is used for risk stratification *in vitro.* In an alternative embodiment, said kit is used for the disease severity *in vitro.* In an alternative embodiment, said kit is used for decreased oxygen in blood (hypoxia) *in vitro.* In an alternative embodiment, said kit is used for silent hypoxia *in vitro,* where the patient and physicians might not have been aware of the hypoxia and disease severity based on clinical symptoms. In an alternative embodiment, said kit is used for under perfusion (ischemia), insufficient oxygen supply and necrosis of tissue (generic hypoxia) *in vitro.*

The instructions shall contain a protocol for carrying out the method of predicting the disease severity as described herein above. Further, the instructions shall contain at least one value for a reference for a GDF-15 peptide and optionally at least one value for a reference for TnT-hs peptide.

The "assay" is preferably a kit adapted for determining the level of the biomarker. The term "kit" is explained herein below. E.g. said kit shall comprise at least one detection agent for a GDF-15 peptide and optionally at least one further agent selected from the group consisting of an agent which specifically binds to a cTnT, NT-proBNP, CRP and D-dimer. The detection agents for the one to five biomarkers can be provided in a single kit or in separate kits.

In a further preferred embodiment of the aforementioned method, GDF-15 is the GDF-15 polypeptide.

In a further preferred embodiment of the aforementioned method, the patient with COVID-19 is 65 years or older.

In a further preferred embodiment of the aforementioned method, the patient with COVID-19 has diabetes mellitus.

In a further preferred embodiment of the aforementioned method, the patient with COVID-19 has a lower oxygen saturation.

In a further preferred embodiment of the aforementioned method, the patient with COVID-19 has a higher National Warning Score at admission.

**In further embodiments, the present invention relates to the following items:**
1. A method for predicting the disease severity in a patient with Betavirus infection, said method comprising
   a) determining the level of GDF-15 in a sample from the patient with Betavirus infection,
   b) comparing the level or concentration determined in step a) to a reference, and
   c) predicting the disease severity in a patient with Betavirus infection.
2. A method for predicting the disease severity in a patient with COVID-19, said method comprising
   a) determining the level of GDF-15_in a sample from the patient with COVID-19,
   b) comparing the level determined in step a) to a reference, and
   c) predicting the disease severity in a patient with COVID-19.
3. The method of aspects 1, wherein an increased level of GDF-15 is indicative for an increased disease severity of a patient with COVID-19.
4. A method for risk stratification of a patient with Betavirus infection, said method comprising
   a) determining the level of GDF-15 in a sample from the patient with Betavirus infection,
   b) comparing the level determined in step a) to a reference,
   c) stratifying the risk of a patient with Betavirus infection.
5. A method for risk stratification of a patient with COVID-19, said method comprising
   a) determining the level of GDF-15 in a sample from the patient with COVID-19,
   b) comparing the level determined in step a) to a reference,
   c) stratifying the risk of a patient with COVID-19.
6. The method of aspects 1 to 5, wherein patients with an increase level of GDF-15 are stratified as having an increased risk for a more sever disease progression.
7. A method for monitoring disease progression in a patient with Betavirus infection, said method comprising
   a) determining the level of GDF-15 in a first sample from the patient with Betavirus infection,
   b) determining the level of GDF-15 in a second sample from the patient with Betavirus infection which has been obtained after the first sample, and
   c) comparing the level of GDF-15 in the first sample to the level of GDF-15 in the second sample, and
   d) monitoring disease progression in a patient with Betavirus infection, based on the results of step c).
8. A method for monitoring disease progression in a patient with COVID-19, said method comprising
   a) determining the level of GDF-15 in a first sample from the patient with COVID-19,
   b) determining the level of GDF-15 in a second sample from the patient with COVID-19 which has been obtained after the first sample, and
   c) comparing the level of GDF-15 in the first sample to the level of GDF-15 in the second sample, and
   d) monitoring disease progression in a patient with COVID-19, based on the results of step c).
9. A method according to aspect 7 and 8, wherein the second sample is taken at a later time point than the first sample.
10. A method according to aspects 7 or 9, wherein the time interval between taking the first and the second sample is at least 3 hours, 1 day, 3 days, 10 or 20 days
11. A method according to aspects 7 to 10, wherein additional samples (third, fourth, fifth sample) are taken at a later time point, wherein the time interval between taking the next sample is at least 3 hours, 1 day, 3 days, 10 or 20 days after the prior sample
12. A method according to aspects 7 to 11, wherein an increased level of GDF-15 in the second sample is indicative of a more severe disease progression.
13. A method according to aspects 7 to 12, wherein a patient with an increasing GDF-15 value is monitored more closely.
14. A method according to aspects 7 to 13, wherein the first sample from the patient with COVID-19 is taken ad admission into hospital, the second sample is taken at day 3, and a third sample is taken at day 9.
15. A method of predicting the risk in a patient with Betavirus infection for hospital admission, said method comprising
   a) determining the level of GDF-15 in a sample from the patient with Betavirus infection,
   b) comparing the level determined in step a) to a reference,
   c) predicting the risk for hospital admission in a patient with Betavirus infection.
16. A method of predicting the risk in a patient with COVID-19 for hospital admission,
   said method comprising
   a) determining the level of GDF-15 in a sample from the patient with COVID-19,
   b) comparing the level determined in step a) to a reference,
   c) predicting the risk for hospital admission in a patient with COVID-19.
17. A method according to aspect 16, wherein hospital admission is recommended for a patient with an increased GDF-15 level.
18. A method for prediction the need for intensive care of a patient with Betavirus infection said method comprising
   a) determining the level of GDF-15 in a sample from the patient with Betavirus infection
   b) comparing the level determined in step a) to a reference,
   c) predicting the need for intensive care of a patient with Betavirus infection.
19. A method for prediction the need for intensive care of a patient with COVID-19, said method comprising
   a) determining the level of GDF-15 in a sample from the patient with COVID-19,
   b) comparing the level determined in step a) to a reference,
   c) predicting the need for intensive care of a patient with COVID-19.
20. A method according to aspect 19, wherein an increased level of GDF-15 is indicative for the need for intensive care of a patient with COVID-19.
21. A method for selecting a patient with Betavirus infection for admission or treatment at Intensive Care Unit, said method comprising
   a) determining the level of GDF-15 in a sample from the patient with Betavirus infection
   b) comparing the level or concentration determined in step a) to a reference,
   c) selecting a patient with Betavirus infection for an Intensive Care Unit.
22. A method for selecting a patient with COVID-19 for admission or treatment at Intensive Care Unit, said method comprising
   a) determining the level of GDF-15 in a sample from the patient with COVID-19,
   b) comparing the level determined in step a) to a reference,
   c) selecting a patient with COVID-19 for an Intensive Care Unit.
22. A method according to aspect 21, wherein patients with increased level of GDF-15 are selected for ICU admission or treatment.
23. A method according to aspects 21 and 22, wherein patients selected for ICU are expected to have complications of their clinical outcome within 27 and 72 hours.
24. A method according to aspects 21 to 23, wherein a patient with a GDF-15 level in the second, third and fourth quartiles is recommended for Intensive Care Unit.
25. A method according to aspects 21 to 24, wherein a patient with a GDF-15 level in the fourth quartile of a COVID-19 population is classified as severe and/or critical and recommended for Intensive Care Unit.
26. A method according to aspects 21 to 25, wherein the upper quartile of GDF-15 has been derived from a representative COVID-19 cohort and is preferentially at least 3000 pg/mL, 4000 pg/mL or 4500 pg/mL.
27. A method according to aspects 21 to 26, wherein the first sample from the patient with COVID-19 is obtained to take in-hospital decisions for regular or intensive care unit (ICU).
28. A method according to aspects 21 to 27, wherein the first sample from the patient with COVID-19 is obtained at admission to the ICU.
29. A method according to aspects 21 to 28, wherein the second sample from the patient with COVID-19 is obtained at admission to the ICU or at day 1 (24 hours), 2 (72 hours), 3 after admission to the ICU.
30. A method of method for predicting the risk for mortality in a patient with Betavirus infection, said method comprising
   a) determining the level of GDF-15 in a sample from the patient with Betavirus infection,
   b) comparing the level determined in step a) to a reference,
   c) predicting the risk for mortality in a patient with Betavirus infection.
31. A method for predicting the risk for mortality in a patient with COVID-19,
   said method comprising
   a) determining the level of GDF-15 in a sample from the patient with COVID-19,
   b) comparing the level determined in step a) to a reference,
   c) predicting the risk for mortality in a patient with COVID-19.
32. A method according to aspect 31, wherein an increased level of GDF-15 is indicative for an increased risk for mortality in a patient with COVID-19.
33. A method according to aspects 31 to 32, wherein a GDF-15 level in the third and fourth quartiles is associated with a higher risk of mortality.
34. A method for predicting the risk for thrombosis in a patient with Betavirus infection, said method comprising
   a) determining the level of GDF-15 in a sample from the patient with Betavirus infection,
   b) comparing the level determined in step a) to a reference,
   c) predicting the risk for thrombosis of a patient with Betavirus infection.
35. Method for predicting the risk for thrombosis in a patient with COVID-19,
   said method comprising
   a) determining the level of GDF-15 in a sample from the patient with COVID-19,
   b) comparing the level determined in step a) to a reference,
   c) predicting the risk for thrombosis of a patient with COVID-19.
36. A method according to aspect 35, wherein an increased level of GDF-15 is indicative for an increased risk of thrombosis in a patient with COVID-19.
37. A method for predicting the risk for pulmonary embolism of a patient with Betavirus infection, said method comprising
   a) determining the level of GDF-15 in a sample from the patient with Betavirus infection,
   b) comparing the level determined in step a) to a reference,
   c) predicting the risk for pulmonary embolism of a patient with Betavirus infection.
38. Method for predicting the risk for pulmonary embolism of a patient with COVID-19, said method comprising
   a) determining the level of GDF-15 in a sample from the patient with COVID-19,
   b) comparing the level determined in step a) to a reference,
   c) predicting the risk for pulmonary embolism of a patient with COVID-19.
39. A method according to aspect 38, wherein an increased level of GDF-15 is indicative for an increased risk for pulmonary embolism of a patient with COVID-19.
40. A method according to accepts 30 to 31, wherein an increased level of GDF-15 is indicative for the increased need of mechanical ventilation and prevention of hypoxia.
41. A method according to aspects 1 to 40, wherein the sample from the patient is body fluid sample.
42. A method according to aspects 1 to 41, wherein the sample is a whole blood, serum or plasma sample.
43. A method for a computer-implemented method for predicting the disease severity in a patient with Betavirus infection, said method comprising
   a) receiving at a processing unit a value for the level of GDF-15 in a sample from a patient with Betavirus infection,
   b) processing the value received in step (a) with the processing unit, wherein said processing comprises retrieving from a memory one or more threshold values for the level of GDF-15 and comparing the value received in step (a) with the one or more threshold values, and
   c) providing a prediction of the disease severity of a patient with Betavirus infection via an output device, wherein said prediction is based on the results of step (b).
44. A computer-implemented method for predicting the disease severity in a patient with COVID-19, said method comprising
   a) receiving at a processing unit a value for the level of GDF-15 in a sample from a patient with COVID-19,
   b) processing the value received in step (a) with the processing unit, wherein said processing comprises retrieving from a memory one or more threshold values for the level of GDF-15 and comparing the value received in step (a) with the one or more threshold values, and
   c) providing a prediction of the disease severity of a patient with COVID-19 via an output device, wherein said prediction is based on the results of step (b).
45. *In vitro* use of GDF-15 or of an agent which binds to GDF-15 for
   a) predicting the disease severity of a patient with Betavirus infection,
   b) risk stratification of a patient with Betavirus infection,
   c) monitoring the extent of the disease severity of a patient with Betavirus infection.
46. *In vitro* use of GDF-15 or of an agent which binds to GDF-15 for
   a) predicting the disease severity of a patient with COVID-19,
   b) risk stratification of a patient with COVID-19,
   c) monitoring the extent of the disease severity of a patient with COVID-19.
47. The method of any one of items 1 to 36, or the *in vitro* use of item 33, wherein
   (1) GDF-15 is the GDF-15 polypeptide,
   (2) the patient with COVID-19 is 65 years or older,
   (3) the patient with COVID-19 has diabetes mellitus,
   (4) the patient with COVID-19 has a lower oxygen saturation, and/or
   (5) the patient with COVID-19 has a higher National Warning Score at admission.
48. The method of any one of claims 1 to 47, or the *in vitro* use of claims 45 to 46, wherein the silent hypoxia and insufficient tissue oxygenation is clinically not visible ("asymptomatic").
49. The method of any one of claims 1 to 48, or the *in vitro* use of claim 45 to 46, wherein the hypoxia and insufficient tissue oxygenation of the patient with Betavirus infection is clinically not visible ("asymptomatic", or "silent hypoxia").
50. The method of any one of claims 1 to 49, or the *in vitro* use of claim 45 to 46, wherein the hypoxia and insufficient tissue oxygenation of the patient with COVID-19 is clinically not visible ("asymptomatic", or "silent hypoxia").

### Examples

The following examples and figures are provided to aid the understanding of the present invention, the true scope of which is set forth in the appended claims. It is understood that modifications can be made in the procedures set forth without departing from the spirit of the invention.

### Background

Growth differentiation factor 15 (GDF-15) is a strong prognostic marker in sepsis and cardiovascular disease (CVD). The prognostic importance of GDF-15 in COVID-19 is unknown.

### Methods

Consecutive, hospitalized patients with laboratory-confirmed infection with severe acute respiratory syndrome coronavirus 2 (SARS-CoV-2) and symptoms of COVID-19 were enrolled in the prospective, observational COVID MECH study. Biobank samples were collected at admission, day 3 and day 9. The primary endpoint was admission to the intensive care unit or death during hospitalization. Linear and logistic regression models were used to assess the prognostic value of biomarkers at baseline, and changes during hospitalization.

### Results

Of the 123 patients enrolled, 35 (28%) reached the primary endpoint; these patients were older, more often had diabetes mellitus, had lower oxygen saturations and higher National Early Warning Score at admission. Admission GDF-15 concentrations were elevated (>95^{th} percentile in age-stratified healthy individuals) in 97 (79%), and higher concentrations were associated with detectable SARS-CoV-2 viremia. Patients reaching the primary endpoint had higher concentrations of GDF-15 (median 4225 [IQR 3197-5972] pg/mL vs 2187 [1344-3620] pg/mL, p<0.001), with C-statistics 0.78 (95% confidence interval 0.70-0.86). The association between GDF-15 and outcome persisted after adjusting for age, sex, race, BMI, CVD and creatinine (p<0.001), and was superior and incremental to interleukin-6, C-reactive protein, procalcitonin, ferritin, D-dimer, cardiac troponin T and N-terminal pro-B-type natriuretic peptide. Increase in GDF-15 from admission to day 3 was also greater in patients reaching the primary endpoint (median 1208 [IQR 0-4305] pg/mL versus -86 [IQR -322-491] pg/mL, p<0.001).

### Patient population

The COVID Mechanisms (COVID MECH) Study is a prospective, observational study with a dedicated biobank enrolling consecutive patients hospitalized with COVID-19. All patients ≥18 years of age hospitalized with laboratory-confirmed COVID-19 at Akershus University Hospital were invited to participate in the study. The institution is a tertiary care center with the largest hospital catchment area in Norway, 560,000 individuals. COVID-19 was defined by a positive SARS-CoV-2 real-time polymerase chain reaction (RT-PCR) nasopharyngeal swab and COVID-19 symptoms as the main reason for admission. The enrolment period was between March 18^{th} and May 4^{th} 2020, covering the majority of the first wave of the pandemic in Norway. The study was registered at ClinicalTrials.gov (NCT04314232) prior to inclusion of patients. Study-specific consent forms were signed by all participants, or by the next-of-kin if the patient was unable to consent (i.e., on invasive mechanical ventilation).

The study was approved by the Regional Ethics Committee of Norway (REK South-East C, Ref. no. 117589) and by the institutional Data Protection Officer (Ref.no. 20/02873).

### Clinical Data collection

Information on clinical characteristics, medical history, detailed COVID-19 presentation, treatments, complications and outcomes were extracted from electronic medical records by the investigators. The primary endpoint in the COVID MECH study was the composite of admission to the intensive care unit (ICU) and/or hospital mortality. Participants were classified as ICU-patients if they were admitted to the ICU ward at any point during their admission and received intensive care treatment >24 hours.

CVD was defined as a history of previous myocardial infarction, heart failure or atrial fibrillation. Obesity was defined as BMI ≥30 kg/m². National Early Warning Scores (NEWS) were calculated from measurements at admission in the emergency department: respiratory rate, oxygen saturation, systolic blood pressure, pulse rate, temperature and level of consciousness.

### Blood sampling procedures and laboratory analysis

Blood samples were drawn by venipuncture at three pre-specified time points: at admission (in the emergency department), day 2-5 of hospitalization (target day 3) and day 6-12 of hospitalization (target day 9). Collection of blood samples was performed by trained nurses at the individual clinical departments. Samples analyzed at the central laboratory were analyzed immediately. Samples in the biobank were temporarily stored a 4°C, centrifuged at 2000 G for 10 minutes and then transferred into aliquots that were frozen and stored at - 80°C at Akershus University Hospital.

The following biomarkers were systematically measured by the central laboratory at Akershus University Hospital in blood samples from all patients admitted with COVID-19: Hemoglobin, white blood cell count (WBC), lymphocyte count, thrombocyte count, D-dimer, C-reactive protein (CRP), sodium, potassium, creatinine, alanine transaminase (ALAT), bilirubin, lactate dehydrogenase (LD) and lactate.

Serum samples from the biobank that had not been previously thawed were used to measure IL-6, procalcitonin (PCT), ferritin, cardiac troponin T (cTnT), N-terminal pro-B-type natriuretic peptide (NT-proBNP) and GDF-15. These biomarkers were analyzed by the electrochemiluminescence immunoassay Elecsys on the Cobas e 801 platform (Roche Diagnostics, Penzberg, Germany). For GDF-15, the CVs reported from the manufacturer are 1.3% at 472 pg/mL and 1.1% at 19368 pg/ml. For IL-6 the coefficient of variations (CVs) reported from the manufacturer are 4.9% at 6.4 pg/mL and 1.4% at 189 pg/ml. For PCT the CVs reported from the manufacturer are 6.9% at 0.12 ng/mL and 1.8% at 43.3 ng/ml. For ferritin, the CVs reported from the manufacturer are 1.5% at 414 ng/mL and 2.8% at 1406 ng/ml. For cTnT, the CVs reported from the manufacturer are 3.5% at 9.7 ng/L and 2.1% at 19.8 ng/L. For NT-proBNP, the CVs reported from the manufacturer are 2.5% at 127 ng/L and 1.3% at 1706 ng/L.

As upper reference limit (URL) we used the 95^{th} percentile concentration of GDF-15 in healthy volunteers (reported from the manufacturer):
831 pg/mL for <30 years, 852 pg/mL for 30-40 years, 1229 pg/mL for 40-50 years, 1466 pg/mL for 50-60 years, 1476 pg/mL for 60-70 years and 2199 pg/mL for ≥70 years. We used 14 ng/L as the URL for cTnT, 125 ng/L and 450 ng/L as the URL for NT-proBNP in participants <75 and ≥75 years, respectively, and 0.5 mg/L as the URL for D-dimer. For SARS-CoV-2 viremia analyses, total nucleic acids were extracted from 200 µl plasma on the MagNA Pure 96 system (Roche, Penzberg, Germany), with an elution volume of 50 µl. SARS-CoV-2 RNA was detected by real-time RT-PCR on a QuantStudio^{™} 7 PCR system (Thermofisher Scientific, Waltham, Massachussets, USA), according to the protocol of Corman et al targeting the viral E-gene.

Patients were classified with viremia if SARS-CoV-2 RNA was detected.

### Statistical analysis

Values are reported as N (%) and median (quartile 1 to quartile 3) for skewed and mean ± SD for normally distributed variables, if not stated otherwise. Categorical and continuous variables were compared using chi-squared test for binary variables, ANOVA for parametric continuous variables, and Kruskal-Wallis for non-parametric continuous variables. Clinical factors associated with log-transformed baseline GDF-15 levels were determined using linear regression including age, sex, race, CVD, body mass index (BMI), systolic blood pressure, heart rate, body temperature, respiratory rate, and log-transformed creatinine, IL-6, CRP, PCT, ferritin, D-dimer, cTnT and NT-proBNP.

Correlations between biomarkers were assessed with the Spearman test. Linear and logistic regression models were used to explore the association between SARS-CoV-2 viremia at admission and log-transformed levels of GDF-15 and other biomarkers. The association between log-transformed admission biomarker concentrations and the primary endpoint was examined in unadjusted and two adjusted logistic regression models: Model 1 adjusted for age, sex, race, CVD, BMI, and log-transformed serum creatinine. Model 2 adjusted for all variables in Model 1 in addition to IL-6, CRP, PCT, ferritin, D-dimer, cTnT and NT-proBNP concentrations.

Area under the receiver operating curves (ROC AUC) were calculated to assess the performance of each biomarker to discriminate between patients reaching and patients not-reaching the endpoint. Changes in biomarkers from admission to day 3 were assessed by the Wilcoxon matched-pairs signed-rank test. Association between changes in biomarkers from admission to day 3 and the primary endpoint was assessed using the same models as for admission values, described above, with additional adjustment for baseline values to account for potential regression to the mean. All delta values used in regression models were calculated from log-transformed baseline and day 3 concentrations.

All statistical analyses were performed using Stata Software (version 16, Stata Corp., College Station, TX, USA). A two-sided *P*-value of <0.05 was considered statistically significant.

### RESULTS

### Baseline characteristics

In total, 123 of 136 consecutive patients hospitalized with COVID-19 in the study period had blood samples collected in the COVID MECH biobank. The mean age was 59.6 ± 15.2 (range 25-87) years, 72 (59%) were men and 68 (55%) were white. Overall, 75 (61%) had one or more comorbidity, including 39 (32%) with hypertension, 35 (29%) obese, 21 (17%) with CVD, 21 (17%) diabetes, and 6 (5%) with COPD. Time from symptom start to hospitalization was 10 ±7 days; 100 (81%) had fever, 98 (80%) had cough and 86 (70%) had dyspnea. At admission the mean body temperature was 38.1 ±0.9 °C, respiratory rate 27 ±9 /min, systolic blood pressure 132 ± 19 mmHg, oxygen saturation 93 ±6 %, and NEWS 5 ±3 points.

### Outcome during hospital stay

During hospitalization, 31 patients were admitted to the ICU. Among these, 27 patients were treated with invasive mechanical ventilation and 4 patients died. The remaining 88 patients were treated in medical wards, and among these 4 patients with treatment restrictions (i.e., do not resuscitate orders) died. Patients who were admitted to the ICU or died (primary study endpoint, n=35) were older, more often had diabetes mellitus, and presented with higher baseline temperature, respiratory rate, NEWS-score and lower oxygen saturations **(Table 1).**

**Table 1 Baseline characteristics of the total COVID MECH study population (n=123) stratified by the primary study endpoint (admission to the intensive care unit or death).**

| | **Non-ICU survivor** | | **ICU or death** | | |
|---|---|---|---|---|---|
| | **n=88** | | **n=35** | | **P-value** |
| Age, years | 57.8 ± 16.3 | | 64.3 ± 10.7 | | 0.031 |
| Male sex | 47 | (53.4%) | 25 | (71.4%) | 0.07 |
| White race | 47 | (53.4%) | 21 | (60.0%) | 0.51 |
| Body mass index, kg/m² | 28.2 ± 5.3 | | 28.5 ± 6.2 | | 0.79 |
| Obesity | 24 | (27.3%) | 11 | (31.4%) | 0.64 |
| Diabetes Mellitus | 11 | (12.5%) | 10 | (28.6%) | 0.033 |
| Hypertension | 25 | (29.1%) | 14 | (40.0%) | 0.24 |
| Cardiovascular disease | 11 | (12.5%) | 7 | (20.0%) | 0.29 |
| Chronic pulmonary disease | 5 | (5.7%) | 1 | (2.9%) | 0.51 |
| Smoking | 4 | (4.5%) | 2 | (5.7%) | 0.79 |
| RAS inhibitors | 23 | (26.1%) | 12 | (34.3%) | 0.37 |

| **Symptoms** | | | | | |
|---|---|---|---|---|---|
| Days of symptoms | 9.9 ± 7.4 | | 9.7 ± 5.6 | | 0.89 |
| Fever | 70 | (79.5%) | 30 | (85.7%) | 0.43 |
| Cough | 71 | (80.7%) | 27 | (77.1%) | 0.66 |
| Dyspnea | 58 | (65.9%) | 28 | (80.0%) | 0.12 |

| **Parameters at admission** | | | | | |
|---|---|---|---|---|---|
| Temperature, °C | 38.0 ± 0.9 | | 38.4 ± 0.9 | | 0.025 |
| Heart rate, /min | 90.1 ± 17.2 | | 90.2 ± 15.5 | | 0.98 |
| Respiratory rate, /min | 25.3 ± 7.3 | | 30.5 ± 10.5 | | 0.003 |
| Systolic blood pressure, mmHg | 133.5 ± 17.1 | | 128.3 ± 21.9 | | 0.16 |
| Oxygen saturation, % | 94.1 ± 3.2 | | 89.7 ± 8.6 | | <0.001 |
| NEWS score | 4.0 [2.5, 6.0] | | 8.0 [6.0, 10.0] | | <0.001 |

| **Routine blood samples at admission** | | | | | |
|---|---|---|---|---|---|
| Hemoglobin, g/dl | 14.0 [12.9, 14.8] | | 13.2 [12.2, 15.0] | | 0.33 |
| White blood cell count, x 10⁹/L | 5.8 [4.4, 7.8] | | 7.5 [5.7, 9.9] | | 0.016 |
| Lymphocyte count, x 10⁹/L | 1.0 [0.7, 1.4] | | 0.9 [0.7, 1.1] | | 0.52 |
| Thrombocyte count, x 10⁹/L | 185 [147, 247] | | 201.0 [150, 275] | | 0.35 |
| Sodium, mmol/L | 136 [135, 138] | | 136.0 [135, 138] | | 0.99 |
| Potassium, mmol/L | 4.1 [3.7, 4.3] | | 4.2 [3.8, 4.5] | | 0.19 |
| Creatinine, umol/L | 73.0 [58, 89] | | 79.0 [69, 101] | | 0.029 |
| Alanine transaminase, IU/L | 30.5 [23.0, 42.0] | | 37.5 [25.0, 58.0] | | 0.11 |
| Bilirubin, umol/L | 11.5 [9.0, 15.5] | | 13.0 [10.0, 17.0] | | 0.17 |
| Lactate dehydrogenase, U/L | 280.0 [220, 350] | | 390.0 [290, 520] | | <0.001 |
| Lactate, mmol/L | 0.9 [0.7, 1.1] | | 1.1 [0.8, 1.3] | | 0.024 |

| | | | | | |
|---|---|---|---|---|---|
| Abbreviations: RAS = Renin-Angiotensin-System; NEWS = National Early Warning Score | | | | | |

Patients who were admitted to the ICU or died also had higher white blood cell count, creatinine, lactate dehydrogenase and lactate at admission. There was no difference in sex, race, BMI, hypertension, underlying cardio vascular disease or chronic pulmonary disease, smoking, days of symptoms, symptoms, or use of renin-angiotensin-system (RAS)-inhibitors with regards to the primary endpoint.

### GDF-15 and other cardiovascular and inflammatory biomarkers at hospital admission

At admission, the median (IQR) concentration was 2798 (1667-4528) pg/mL for GDF-15, 40 (20-75) pg/mL for IL-6, 60 (30-130) mg/L for CRP, 1.4 (0.7-2.3) ug/L for PCT, 477 (238-985) ug/L for ferritin, 0.5 (0.3-1.0) mg/L for D-dimer, 9 (5-16) ng/L for cTnT and 100 (36-285) ng/L for NT-proBNP. CVD biomarker concentrations above the URL was present at admission in 38 (31%) for cTnT, 45 (37%) for NT-proBNP, 72 (61%) for D-dimer, and 97 (79%) for GDF-15.

Table 2: Patients with supra-median concentrations of GDF-15 were older, more often had diabetes mellitus, hypertension and cardiovascular disease, and had higher levels of IL-6, CRP, PCT, Ferritin, cTnT, NT-proBNP, creatinine, LD, bilirubin, D-dimer, WBC, and lower levels of hemoglobin.

**Table 2 shows baseline characteristics stratified by median growth differentiation factor 15 (GDF-15) concentration in the total population (2798 pg/ml).**

| | **Infra-median GDF-15** | | **Supra-median GDF-15** | | **P-value** |
|---|---|---|---|---|---|
| | **n=62** | | **n=61** | | |
| *GDF-15* | *1707 [1170, 2273]* | | *4528 [3673, 7333]* | | |
| Age, years | 54.5 ± 14.9 | | 64.9 ± 13.7 | | <0.001 |
| Male sex | 33 | (53.2%) | 39 | (63.9%) | 0.23 |
| White race | 32 | (51.6%) | 36 | (59.0%) | 0.41 |
| Body mass index, kg/m² | 27.9 ± 4.2 | | 28.7 ± 6.5 | | 0.45 |
| Obesity | 18 | (29.0%) | 17 | (27.9%) | 0.89 |
| Diabetes Mellitus | 2 | (3.2%) | 19 | (31.1%) | <0.001 |
| Hypertension | 11 | (18.0%) | 28 | (46.7%) | <0.001 |
| Cardiovascular disease | 4 | (6.5%) | 17 | (27.9%) | 0.002 |
| Chronic pulmonary disease | 1 | (1.6%) | 5 | (8.2%) | 0.09 |
| Smoking | 4 | (6.5%) | 2 | (3.3%) | 0.41 |

| **Parameters on admission** | | | | | |
|---|---|---|---|---|---|
| Days of symptoms | 10.6 ± 7.3 | | 9.2 ± 6.5 | | 0.25 |
| Temperature, °C | 38.15 ± 0.85 | | 38.03 ± 0.96 | | 0.47 |
| Heart rate, /min | 88.6 ± 15.0 | | 91.7 ± 18.2 | | 0.30 |
| Respiratory rate, /min | 25.3 ± 7.7 | | 28.3 ± 9.3 | | 0.05 |
| Systolic blood pressure, mmHg | 133.8 ± 17.0 | | 130.3 ± 20.1 | | 0.29 |

| **Laboratory values** | | | | | |
|---|---|---|---|---|---|
| Hemoglobin, g/dL | 14.2 [13.2, 15.0] | | 13.4 [12.1, 14.3] | | 0.008 |
| White blood cell count, x 10⁹ /L | 5.2 [4.1, 7.5] | | 7.4 [5.5, 9.7] | | <0.001 |
| Lymphocyte count, x 10⁹ /L | 1.0 [0.8, 1.5] | | 0.9 [0.7, 1.1] | | 0.10 |
| Thrombocyte count, x 10⁹ /L | 181 [140, 236] | | 189 [156, 254] | | 0.22 |
| Sodium, mmol/L | 136 [135, 138] | | 136 [134, 138] | | 0.34 |
| Potassium, mmol/L | 4.1 [3.7,4.2] | | 4.2 [3.8, 4.5] | | 0.05 |
| Creatinine, umol/L | 68 [56, 82] | | 84 [70, 114] | | <0.001 |
| Alanine transaminase, IU/L | 30 [21, 41] | | 37 [26, 54] | | 0.14 |
| Bilirubin, umol/L | 11 [8, 14] | | 13 [10, 17] | | 0.028 |
| Lactate dehydrogenase, U/L | 260 [210, 300] | | 360 [270, 500] | | <0.001 |
| Lactate, mmol/L | 0.9 [0.7, 1.1] | | 1.0 [0.8, 1.3] | | 0.09 |
| Interleukin-6, pg/mL | 22.6 [13.8, 46.5] | | 59.9 [38.3, 106.0] | | <0.001 |
| C-reactive protein, mg/L | 35.0 [20.0, 90.0] | | 100.0 [60.0, 190.0] | | <0.001 |
| Procalcitonine, ug/L | 0.7 [0.5, 1.4] | | 2.1 [1.4, 5.1] | | <0.001 |
| Ferritin, ug/L | 295 [176, 643] | | 710 [438, 1580] | | <0.001 |
| D-dimer, mg/L | 0.4 [0.3, 0.7] | | 0.8 [0.5, 1.3] | | <0.001 |
| cTnT, ng/L | 6.0 [4.0, 11.0] | | 13.0 [8.0, 26.0] | | <0.001 |
| NT-proBNP, ng/L | 49.5 [23.0, 126.0] | | 169.0 [97.0, 480.0] | | <0.001 |

There were no differences in duration of symptoms or symptom characteristics by GDF-15 concentrations. In multivariable regression models, higher GDF-15 concentration was associated with older age, non-white race, and higher PCT and D-dimer concentrations **(Table 3).**

**Table 3 Multivariable regression of admission growth differentiation factor 15 concentrations**

| | **Coef. (95% CI)** | **P-value** |
|---|---|---|
| Age, years | 0.015 (0.004 to 0.026) | 0.009 |
| Male sex | | 0.78 |
| White race | -0.27(-0.53 to -0.02) | 0.04 |
| Body mass index, kg/m² | | 0.95 |
| Cardiovascular disease | | 0.43 |
| Temperature, °C | | 0.19 |
| Heart rate, /min | | 0.16 |
| Respiratory rate, /min | | 0.31 |
| Systolic blood pressure, mmHg | | 0.12 |
| Oxygen saturation, % | | 0.37 |
| Creatinine, umol/L | | 0.13 |
| Interleukin-6, pg/mL | | 0.62 |
| C-reactive protein, mg/L | | 0.17 |
| Procalcitonine, ug/L | 0.16(0.003 to 0.32) | 0.046 |
| Ferritin, ug/L | | 0.87 |
| D-dimer, mg/L | 0.20(0.07 to 0.34) | 0.004 |
| TnT, ng/L | | 0.93 |
| NT-proBNP, ng/L | | 0.60 |

| | | |
|---|---|---|
| Abbreviations: IL-6= interleukin 6; CRP = C-reactive protein; PCT = procalcitonine; cTnT = cardiac troponin T; NT-proBNP = N-terminal pro-B-type natriuretic peptid. | | |

GDF-15 correlated with all the other inflammatory and cardiovascular biomarkers; correlation coefficients ranging from 0.45 to 0.64 (Table 4).

**Table 4 Spearman correlation between admission concentrations of biomarkers**

| | **GDF-15** | **IL-6** | **CRP** | **PCT** | **Ferritin** | **D-Dimer** | **cTnT** | **NT-proBNP** |
|---|---|---|---|---|---|---|---|---|
| **IL-6** | 0.50 | | | | | | | |
| **CRP** | 0.53 | 0.62 | | | | | | |
| **PCT** | 0.64 | 0.69 | 0.65 | | | | | |
| **Ferritin** | 0.45 | 0.49 | 0.54 | 0.67 | | | | |
| **D-Dimer** | 0.46 | 0.29 | 0.47 | 0.37 | 0.36 | | | |
| **cTnT** | 0.52 | 0.40 | 0.31 | 0.43 | 0.25 | 0.32 | | |
| **NT-proBNP** | 0.49 | 0.50 | 0.42 | 0.51 | 0.34 | 0.41 | 0.68 | |
| **Creatinine** | 0.42 | 0.27 | 0.30 | 0.47 | 0.30 | 0.22 | 0.62 | 0.42 |

| | | | | | | | | |
|---|---|---|---|---|---|---|---|---|
| All correlations were significant (p<0.05) Abbreviations: IL-6= interleukin 6; CRP = C-reactive protein; PCT = procalcitonine; cTnT = cardiac troponin T; NT-proBNP = N-terminal pro-B-type natriuretic peptide; GDF-15= growth differentiation factor 15 | | | | | | | | |

### GDF-15 and SARS-CoV-2 viremia

SARS-CoV-2 viremia was present in 48 (39%) of patients at admission. Grouping patients by quartiles of GDF-15 concentrations at admission, we found that a higher GDF-15 quartile was associated with a greater proportion of patients having viremia (p<0.001 for trend) **(****Figure 4****).**

Viremia at admission ranged from 13% in the lowest quartile to 60% in the highest quartile of GDF-15. Viremia remained associated with higher quartile of GDF-15, also after adjusting for age, sex, race, BMI, CVD, creatinine (p=0.001). Only GDF-15 was associated with viremia at hospital admission in regression models including IL-6, CRP, PCT, ferritin, cTnT, NT-proBNP and GDF-15 **(Table 5).**

**Table 5 Multivariable logistic regression of severe acute respiratory syndrome coronavirus 2 viremia at admission, with all variables listed in the model.**

| | | **OR (95% CI)** | **P-value** |
|---|---|---|---|
| IL-6 | (pg/mL) | 1.63 (0.97-2.74) | 0.07 |
| CRP | (mg/L) | 1.61 (0.90-2.88) | 0.29 |
| PCT | (ug/L) | 0.72 (0.39-1.31) | 0.11 |
| Ferritin | (ug/L) | 1.58 (0.93-2.68) | 0.09 |
| D-dimer | (mg/L) | 0.60 (0.34-1.06) | 0.08 |
| cTnT | (ng/L) | 1.35 (0.74-2.44) | 0.32 |
| NT-proBNP | (ng/L) | 0.73 (0.48-1.13) | 0.16 |
| GDF-15 | (pg/mL) | 1.96 (0.14-3.35) | 0.014 |

| | | | |
|---|---|---|---|
| All biomarker values were log-2 transformed in the adjusted regression model. Abbreviations: IL-6= interleukin 6; CRP = C-reactive protein; PCT = procalcitonine; cTnT = cardiac troponin T; NT-proBNP = N-terminal pro-B-type natriuretic peptide; GDF-15= growth differentiation factor 1 | | | |

### Association between cardiovascular and inflammatory biomarkers at admission and outcome

Higher admission concentrations of IL-6, CRP, PCT, ferritin, NT-proBNP and GDF-15 were associated with the primary endpoint, while there was no association for cTnT and D-dimer **(****Figure 1****).** IL-6, CRP, PCT, ferritin and GDF-15 remained associated with the primary endpoint after adjusting for baseline characteristics (age, sex, race, BMI, CVD and creatinine).

In regression models including all biomarkers and baseline characteristics, ferritin (p=0.03) and GDF-15 (p=0.006) were the only biomarkers associated with the primary endpoint. The ROC AUC for GDF-15 to discriminate patients with the primary endpoint was 0.78 (95% CI 0.70-0.86) **(****Figure 1****).**

**Figure 1** Concentrations of admission cardiovascular and inflammatory biomarkers and the association with the primary endpoint among patients hospitalized with COVID-19 (n=123).

Model 1: Adjusted for age, sex, race, body mass index, cardiovascular disease and creatinine Model 2: Adjusted for Model 1 + all the other biomarkers in the table Concentrations are reported as median (Q1, Q3). All biomarker values were log-transformed in the adjusted regression models.

Abbreviations: IL-6= interleukin 6; CRP = C-reactive protein; PCT = procalcitonine; cTnT = cardiac troponin T; NT-proBNP = N-terminal pro-B-type natriuretic peptide; GDF-15= growth differentiation factor 15

Non-survivors had higher IL-6, D-dimer, cTnT, NT-proBNP and GDF-15 in unadjusted models **(****Figure 2****).** After adjusting for age, sex, race, BMI, CVD and creatinine, only GDF-15 remained associated with hospital mortality. Median (IQR) GDF-15 was 7789 (4716-9317) pg/mL in non-survivors and 2583 (1512-4225) pg/mL in survivors. The ROC AUC for GDF-15 as a discriminator for hospital mortality was 0.87 (95% CI 0.79-0.94) **Figure 2****).** All of the 8 non-survivors and 25 of 31 (81%) patients admitted to the ICU had GDF-15 concentrations above the median.

**Figure 2** Concentrations of admission cardiovascular and inflammatory biomarkers and the association with all cause death among patients hospitalized with COVID-19 (n=123)
Model 1: Adjusted for age, sex, race, body mass index, cardiovascular disease and creatinine Concentrations are reported as median (Q1, Q3). All biomarker values were log transformed in the adjusted regression model. Abbreviations: IL-6= interleukin 6; CRP = C-reactive protein; PCT = procalcitonine; cTnT = cardiac troponin T; NT-proBNP = N-terminal pro-B-type natriuretic peptide; GDF-15= growth differentiation factor 1.

### Changes in biomarkers during hospitalization versus outcome

Serial samples from admission and day 3 were available in 96 (78%) of patients, and there was a significant increase in CRP, PCT, NT-proBNP, GDF-15 and D-dimer levels from baseline to day 3 in the total population. The primary outcome, admission to the ICU or death, occurred in 33 (34%) of these patients. GDF-15 increased by median 1208 pg/mL (IQR 0-4305] in patients reaching the primary endpoint and decreased by median 85 [IQR -322-491] pg/mL in non-ICU survivors. In unadjusted models, greater increases in IL-6, PCT, D-dimer and GDF-15 from admission to day 3 were associated with the primary endpoint **(****Figure 3****).** IL-6, D-dimer and GDF-15 were still associated with the primary endpoint after adjusting for age, sex, race, BMI, CVD and baseline creatinine. These associations remained significant when adjusting for the admission concentration of each biomarker. The ROC AUC for delta value of GDF-15 was 0.69 (95% CI 0.57-0.81) **(****Figure 3****).**

**Figure 3** Changes in concentrations from admission to day 3 in cardiovascular and inflammatory biomarkers stratified by the primary study endpoint among patients hospitalized with COVID-19 (n=96).

Model 1: Adjusted for age, sex, race, body mass index, cardiovascular disease and creatinine Model 2: Adjusted for Model 1 + the baseline concentration of each biomarker Concentrations are reported as median (Q1, Q3). All biomarker delta values were log-transformed in the adjusted regression models.

Abbreviations: IL-6= interleukin 6; CRP = C-reactive protein; PCT = procalcitonine; cTnT = cardiac troponin T; NT-proBNP = N-terminal pro-B-type natriuretic peptide; GDF-15= growth differentiation factor 15

Changes in biomarker concentrations from admission to day 9 were available in 49 (40%) of the patients, and the primary endpoint occurred in 26 (53%) of these participants. GDF-15 continued to increase to day 9 (median 8031 [IQR 3589-16003] pg/mL in patients reaching the primary endpoint, and remained stable in non-ICU survivors [median 2238 [IQR 1540-3118] pg/mL **(****Figure 2****).**

**Table 6. Changes in concentrations from admission to day 9 in GDF-15 stratified by ICU mortality among patients with COVID-19 (n=31) admitted to the ICU**

| | **ICU survivor (n=27) pg/mL** | **ICU non-survivor (n=4) pg/mL** | **P-value** |
|---|---|---|---|
| **GDF15 Day 1 (n=31)** | 3785.0 [3096.0, 4597.0] | 6497.5 [4189.5, 9237.5] | 0.11 |
| **GDF15 Day 3 (n=29)** | 5092.0 [3681.0, 6824.0] | 14639.5 [6689.0, 20000.0] | 0.10 |
| **GDF15 Day 9 (n=23)** | 7682.0 [3569.5, 14350.5] | 8383.0 [7843.0, 20000.0] | 0.31 |

Changes in biomarker concentrations from admission to day 9 were available also for 31 patients admitted to the ICU. 4 of these participants were observed to be ICU-Non-survivors. GDF-15 concentrations were assessed in ICU non-survivors versus ICU survivors in an exploratory analysis. GDF-15 concentrations were observed to be numerically higher in ICU non-survivors at each time of sampling compared to ICU survivors.

Already at admission to the ICU (Day1) numerically higher GDF-15 concentrations were observed in ICU non-survivors (median 6497.5 [IQR 4189.5-9237.5] versus ICU survivors [median 3785 [IQR 3096-4597] pg/mL (Figure 3). It may be anticipated from the data shown in table 6, that elevated GDF-15 concentrations in patients selected for the ICU may help to early identify patients that will experience complications of their clinical outcome later on within the next 24 and 72 hours of their stay in the ICU.

### DISCUSSION

In this prospective study of consecutive patients hospitalized with COVID-19 elevated admission concentrations of GDF-15 in 80% of patients were found. Higher concentrations of GDF-15 were associated with SARS-CoV-2 viremia on admission, need for intensive care, and hospital mortality. The prognostic performance of GDF-15 was superior to, and independent of, established cardiovascular and inflammatory biomarkers. Increases in GDF-15 from admission to day 3 were associated with worse outcome.

It is an embodiment of the present invention, that GDF-15 concentrations not only predicts the risk, but also has a prognostic value superior to the established risk markers for patients with COVID-19.

Biomarkers of importance in COVID-19 were first identified in Chinese case series of patients hospitalized with COVID-19 in the early phase of the pandemic. These studies reported clinical characteristics and laboratory findings from selected patients, i.e., results were from clinically indicated blood samples. Numerous biomarkers were assessed in association to outcome, and a handful of inflammatory and cardiovascular biomarkers were identified as particularly strong prognostic markers, including cardiac troponins, ferritin and D-dimer. In the present invention, a prospective biobank study of unselected, consecutive patients hospitalized with COVID-19 provides important insights to these associations, since the design alleviates the risk of selection bias. As in previous case series, the inventors of the present invention found IL-6, CRP, PCT, Ferritin, D-dimer, cTnT and NT-proBNP to be associated with worse outcome. However, after adjusting for demographics, BMI, CVD and creatinine, this association was attenuated for IL-6, D-dimer, cTnT and NT-proBNP. Moreover, in models including GDF-15 as well as these biomarkers, only GDF-15 remained associated with ICU admission or death. Based on these findings, GDF-15, which is easily available by commercial assays, may be considered for risk stratification in patients hospitalized with COVID-19.

There were significant changes in inflammatory and cardiovascular biomarkers during hospitalization, with distinct trajectories among patients who were admitted to the ICU or who died. From hospital admission to day 3 there were greater increases in IL-6, D-dimer and GDF-15 in patients with poor outcome, also after adjusting for possible confounders. GDF-15 increased from admission to day 3 by median 1208 pg/mL in patients who were admitted to the ICU or died, while there was a median reduction of 86 pg/mL in non-ICU-admitted survivors. At admission to the ICU (Day1) numerically higher GDF-15 concentrations was observed in ICU non-survivors (median 6497.5) versus ICU survivors (median 3785 pg/mL). Numerically higher GDF-15 concentrations observed at admission in patients selected for ICU may aid early identification of patients experiencing severe complications of their clinical outcome within the 24 or 72 hours in the ICU.

There was a significant association between lower oxygen saturation and higher GDF-15 at baseline: Beta coefficient was -2.23 (95% CI -3.46 to -0.99) per log unit GDF-15, p=0.001. This association persisted after adjusting for age, sex, race, obesity (BMI ≥30 kg/m2), CVD (previous myocardial infarction, heart failure or atrial fibrillation) and creatinine
GDF-15 is known to be a robust predictor of poor outcome in critically ill patients. In animal models, GDF-15 deficiency is known to augment the inflammatory response and to exacerbate renal and cardiac injury induced by lipopolysaccharide, while over-expression of GDF-15 protects from these endotoxin-induced mechanisms. GDF-15 expression is induced by lung injury, and is suggested to be a hallmark for tissue injury in many organs. In the present invention, it was found that SARS-CoV-2 viremia is associated with higher levels of GDF-15. In models including all the cardiovascular and inflammatory biomarkers, only GDF-15 remained associated with viremia. This novel finding may suggest a direct link between SARS-CoV-2 cytopathic effects and the expression of GDF-15 in multiple tissues. GDF-15 correlated moderately (rho 0.45-0.64) with all the investigated inflammatory and cardiovascular biomarkers, but provided prognostic information beyond this. The present invention supports the current, integrated understanding on GDF-15, reflecting a spectrum of pathophysiological effects related to inflammation and cardiovascular dysfunction.

### CONCLUSION

GDF-15 is elevated in 80% of patients hospitalized with COVID-19, and higher concentrations are associated with SARS-CoV-2 viremia and worse clinical outcome. GDF-15 provides prognostic information that is superior to known risk markers in COVID-19, including cTnT, NT-proBNP, CRP and D-dimer. Greater increases in GDF-15 during hospitalization are also independently associated with worse outcome.

## Claims

1. A method for predicting the disease severity in a patient with COVID-19,
said method comprising
a) determining the level of GDF-15 in a sample from the patient with COVID-19,
b) comparing the level determined in step a) to a reference, and
c) predicting the disease severity in a patient with COVID-19.

2. The method of claim 1, wherein an increased level of GDF-15 is indicative for an increased disease severity of a patient with COVID-19.

3. A method for risk stratification of progressing to severe disease of a patient with COVID-19,
said method comprising
a) determining the level of GDF-15 in a sample from the patient with COVID-19,
b) comparing the level determined in step a) to a reference,
c) stratifying the risk of a patient with COVID-19.

4. A method for monitoring disease progression in a patient with COVID-19,
said method comprising
a) determining the level of GDF-15 in a first sample from the patient with COVID-19,
b) determining the level of GDF-15 in a second sample from the patient with COVID-19 which has been obtained after the first sample, and
c) comparing the level of GDF-15 in the first sample to the level of GDF-15 in the second sample, and
d) monitoring disease progression in a patient with COVID-19, based on the results of step c).

5. A method according to claim 4, wherein an increased level of GDF-15 in the second sample is indicative of a more severe disease progression.

6. A method according to any of claims 4 to 5, wherein a patient with an increasing GDF-15 value is monitored more closely.

7. A method of predicting the risk in a patient with COVID-19 for hospital admission, said method comprising
a) determining the level of GDF-15 in a sample from the patient with COVID-19,
b) comparing the level determined in step a) to a reference,
c) predicting the risk for hospital admission in a patient with COVID-19.

8. A method according to claims 6 and 7, wherein patients selected for ICU are expected to have complications of their clinical outcome within the next 24 and 72 hours.

9. A method for prediction the need for intensive care of a patient with COVID-19,
said method comprising
a) determining the level of GDF-15 in a sample from the patient with COVID-19,
b) comparing the level determined in step a) to a reference,
c) predicting the need for intensive care of a patient with COVID-19.

10. A method for selecting a patient with COVID-19 for admission or treatment at Intensive Care Unit, said method comprising
a) determining the level of GDF-15 in a sample from the patient with COVID-19,
b) comparing the level determined in step a) to a reference,
c) selecting a patient with COVID-19 for an Intensive Care Unit.

11. A method according to claim 10, wherein patients with increased level of GDF-15 are selected for ICU admission or treatment.

12. A method according to claims 9 to 11, wherein patients selected for ICU are expected to have complications of their clinical outcome within 24 and 72 hours.

13. A method for predicting the risk for mortality in a patient with COVID-19,
said method comprising
a) determining the level of GDF-15 in a sample from the patient with COVID-19,
b) comparing the level determined in step a) to a reference,
c) predicting the risk for mortality in a patient with COVID-19.

14. A method according to claim 12, wherein an increased level of GDF-15 is indicative for an increased risk for mortality in a patient with COVID-19.

15. Method for predicting the risk for thrombosis in a patient with COVID-19,
said method comprising
a) determining the level of GDF-15 in a sample from the patient with COVID-19,
b) comparing the level determined in step a) to a reference,
c) predicting the risk for thrombosis of a patient with COVID-19.

16. Method for predicting the risk for pulmonary embolism of a patient with COVID-19, said method comprising
a) determining the level of GDF-15 in a sample from the patient with COVID-19,
b) comparing the level determined in step a) to a reference,
c) predicting the risk for pulmonary embolism of a patient with COVID-19.

17. A computer-implemented method for predicting the disease severity in a patient with COVID-19, said method comprising
a) receiving at a processing unit a value for the level of GDF-15 in a sample from a patient with COVID-19,
b) processing the value received in step (a) with the processing unit, wherein said processing comprises retrieving from a memory one or more threshold values for the level of GDF-15 and comparing the value received in step (a) with the one or more threshold values, and
c) providing a prediction of the disease severity of a patient with COVID-19 via an output device, wherein said prediction is based on the results of step (b).

18. *In vitro* use of GDF-15 or of an agent which binds to GDF-15 for
a) predicting the disease severity of a patient with COVID-19,
b) risk stratification of a patient with COVID-19,
c) monitoring the extent of the disease severity of a patient with COVID-19.

19. The method of any one of claims 1 to 18, or the *in vitro* use of claim 18, wherein
I. GDF-15 is the GDF-15 polypeptide,
II. the patient with COVID-19 is 65 years or older,
III. the patient with COVID-19 has diabetes mellitus,
IV. the patient with COVID-19 has a lower oxygen saturation, and/or
V. the patient with COVID-19 has a higher National Warning Score at admission.

20. The method of any one of claims 1 to 19, or the *in vitro* use of claim 17, wherein the hypoxia and insufficient tissue oxygenation of the patient with COVID-19 is clinically not visible ("asymptomatic", or "silent hypoxia")
